(19) Europäisches Patentamt / European Patent Office / Office européen des brevets

(11) **EP 2 067 481 A1**

(12) **EUROPEAN PATENT APPLICATION**

(43) Date of publication:
**10.06.2009 Bulletin 2009/24**

(51) Int Cl.:
*A61K 38/22* (2006.01)     *A61P 3/04* (2006.01)

(21) Application number: **07023373.9**

(22) Date of filing: **03.12.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK RS**<br><br>(71) Applicant: **Charité - Universitätsmedizin Berlin**<br>**10117 Berlin (DE)** | (72) Inventors:<br>• **Kobelt, Peter**<br>**14059 Berlin (DE)**<br>• **Mönnikes, Hubert**<br>**13505 Berlin (DE)**<br><br>(74) Representative: **Engelhard, Markus**<br>**Forrester & Boehmert**<br>**Pettenkoferstrasse 20-22**<br>**80336 München (DE)** |

(54) **Therapeutic use of desacyl ghrelin**

(57)     The present invention refers to desacyl ghrelin and its use in the treatment of the eating disorder hyperphagia. In particular, the present invention refers to a use of a desacyl ghrelin or an active peptide fragment thereof for the preparation of a pharmaceutical composition for the treatment of hyperphagia in a subject. Preferably, hyperphagia is associated with body weight gain and/or obesity, and most preferably, the subject has a body mass index BMI of > 30.

**Fig. 2.**

**Description**

The present invention refers to desacyl ghrelin, in particular to the use of desacyl ghrelin in the treatment of eating disorders, particularly hyperphagia, most particularly hyperphagia associated with obesity.

**Background of the invention**

The gastric peptide ghrelin, an endogenous ligand for growth-hormone secretagogue (GHS) receptor, has two major molecular forms: acylated ghrelin (n-octanoyl-ghrelin, sometimes also briefly referred to as ghrelin) and desacyl ghrelin (des-n-octanoyl ghrelin) (for a review see Kojima and Kangawa 2005). Acylated ghrelin induces a positive energy balance, while desacyl ghrelin was initially thought to be devoid of any endocrine activities. It was speculated that desacyl ghrelin may represent either a preform of ghrelin or the product of its deacylation (Kojima and Kangawa 2005).

Acylated ghrelin is a 28-amino acid peptide hormone characterized by a n-octanoic acid modification at Ser3, which was primarily identified in the rat stomach as the first endogenous ligand of the GHS receptor (Kojima *et al.* 1999). Ghrelin principal peripheral sources of synthesis are the A-like endocrine cells of the gastric oxyntic mucosa (Dornonville *et al.* 2001) which contribute to about 80% of the circulating levels of ghrelin (Kojima *et al.* 1999; Date *et al.* 2000a; Thomas *et al.* 1997; Arvat *et al.* 2001; Asakawa *et al.* 2001a; Nagaya *et al.* 2001). Independent of its endocrine effects (Date *et al.* 2000b; Seoane *et al.* 2000; Tolle *et al.* 2001) peripheral ghrelin has been shown to act as a gut-brain peptide exhibiting a potent orexigenic effect on food intake in rats (Wren *et al.* 2000; Wren *et al.* 2001a; Tschop *et al.* 2000; Nakazato *et al.* 2001), mice (Asakawa *et al.* 2001b; Wang *et al.* 2002), and humans (Wren *et al.* 2001 b).

US 6,967,237 discloses truncated ghrelin analogues active at the GHS receptor. The analogues can bind to the receptor, and preferably, stimulate receptor activity. They have a variety of different uses including being used as a research tool and being used therapeutically. Particularly, the analogues can be used to achieve a beneficial effect in a subject such as facilitating weight gain, maintenance of weight, or appetite increase.

The second form of ghrelin, i.e. desacyl ghrelin, is characterized by the lack of the fatty acid residual at Ser3 (Hosoda *et al.* 2000). This peptide is the main form of total ghrelin in blood circulation; plasma desacyl ghrelin concentration accounts for more than 90% of circulating ghrelin (Hosada *et al.* 2000; Yoshimoto *et al.* 2002). Recent studies indicate that the peptide is involved in the regulation of energy homeostasis in rodents (Asakawa *et a*/. 2005; Chen *et a*/. 2005a). In contrast to acylated ghrelin, it has been reported that desacyl ghrelin exerts an inhibitory effect on food intake after intraperitoneal (ip) or central (intracisternal or intracerebroventricular) administration in rodents (Asakawa *et al.* 2005; Chen *et al.* 2005a). Asakawa *et al.* (2005) reported that the administration of desacyl ghrelin decreased food intake and the gastric emptying rate in normal mice. Further, it has been shown that desacyl ghrelin overepressing mice exhibited a decrease in body weight, food intake, and fat pad mass weight accompanied by moderately decreased linear growth. Gastric emptying was also decreased in desacyl ghrelin overexpressing mice (Asakawa *et al.* 2005).

Most likely, the effect of desacyl ghrelin is through an action on neurons in the paraventricular nucleus (PVN) and arcuate nucleus (ARC) of the hypothalamus (Chen *et al.* 2005b). Most of the mapping of brain neuronal circuits recruited by satiety signals have been obtained by assessing changes in Fos expression, as determined by detection of Fos-immunoreactivity (Fos-ir), which allows to identify activated neurons at the cellular level (Sagar *et al.* 1988). A recent study has shown that peripherally injected desacyl ghrelin activates neurons in the PVN and in the ARC of the hypothalamus which are involved in the regulation of energy homeostasis in mammalian (Asakawa *et al.* 2005; Chen *et al.* 2005a).

Subsequent studies showed that centrally administered desacyl ghrelin stimulates food intake independently from GHS receptor while peripheral administration of peptide had no effects in rodents and humans (Toshinai *et al.* 2006). There is also evidence suggesting that desacyl ghrelin may counteract ghrelin in the control of energy balance and the control of glucose metabolism (Asakawa *et al.* 2005; Gauna *et al.* 2005; Heijboer *et al.* 2006). Some studies showed that the metabolic unlike the neuroendocrine effects of ghrelin can be abrogated by co-administered unacylated ghrelin. We recently reported that simultaneous administration of peripheral cholecystokinin (CCK) or bombesin together with ghrelin blocked the orexigenic action of ghrelin in rats (Kobelt *et al.* 2005; 2006).

WO 03/051389 discloses pharmaceutical compositions containing desacyl ghrelin and derivatives and their uses in the control of glycemia in ageing patients, growth hormone deficient patients, diabetic patients and obese patients. Further disclosed is the use of such compositions for preventing and/or reducing dawn phenomenon in type I diabetes patients or for reducing body weight increased in a patient suffering from type II diabetes and syndrome X.

The object of the present invention is to provide further therapeutic uses of desacyl ghrelin.

**Summary of the invention**

The object of the present invention is solved by a use of a desacyl ghrelin or an active fragment thereof for the preparation of a pharmaceutical composition for the treatment of hyperphagia in a subject in need thereof.

In one embodiment, the desacyl ghrelin comprises a peptide having an amino acid sequence according to any of SEQ ID NO:1 to SEQ ID NO:21.

In one embodiment, the active fragment of desacyl ghrelin comprises a peptide having an amino acid sequence according to any of SEQ ID NO:22 to SEQ ID NO:25.

In one embodiment, hyperphagia is associated with body weight gain and/or obesity.

In one embodiment, hyperphagia is associated with a central nervous disorder.

In one embodiment, hyperphagia is associated with a disorder selected from unipolar depression, bipolar disorder, anxiety, schizophrenia, and binge eating disorder.

In one embodiment, hyperphagia is associated with impaired satiety.

In one embodiment, hyperphagia is associated with a disorder selected from a genetically disposed obesity, Prader-Labhart-Willi-Fanconi syndrome, Laurence-Moon-Biedl syndrome, Fröhlich's syndrome, central Cushing's syndrome, and hyperphagic short stature syndrome.

In a preferred embodiment, the genetically disposed obesity is associated with a MC4R gene mutation.

In one embodiment, the subject has a body mass index BMI of > 30, preferably > 40.

In one embodiment, the subject is an adult.

In one embodiment, the subject is a mammal, preferably a human, most preferably a human in the age of 16 years and above.

In one embodiment, the pharmaceutical composition is comprised by a pharmaceutical dosage form selected from an injection, a tablet, a capsule, a caplet, a suppository, an elixier, an ointment, and a patch.

In a preferred embodiment, the pharmaceutical dosage form is a controlled-release dosage form, preferably a sustained-release dosage form.

The object of the present invention is further solved by a pharmaceutical composition comprising a desacyl ghrelin or an active fragment thereof.

The object of the present invention is further solved by a pharmaceutical composition comprising a desacyl ghrelin or an active fragment thereof for the treatment of hyperphagia in a subject in need thereof.

In one embodiment of the pharmaceutical composition, the desacyl ghrelin comprises a peptide having an amino acid sequence according to any of SEQ ID NO:1 to SEQ ID NO:21.

In one embodiment of the pharmaceutical composition, the active fragment of desacyl ghrelin comprises a peptide having an amino acid sequence according to any of SEQ ID NO:22 to SEQ ID NO:25.

In one embodiment of the pharmaceutical composition, the composition is comprised by a pharmaceutical dosage form selected from an injection, a tablet, a capsule, a caplet, a suppository, an elixier, an ointment, and a patch.

In a preferred embodiment of the pharmaceutical composition, the pharmaceutical dosage form is a controlled-release dosage form, preferably a sustained-release dosage form.

The object of the present invention is further solved by a method of preparation of the pharmaceutical composition.

The object of the present invention is further solved by a method for preparing the pharmaceutical dosage form.

The object of the present invention is further solved by a use of a desacyl ghrelin or an active fragment thereof in the treatment of hyperphagia in a subject in need thereof.

The object of the present invention is further solved by a method of treatment of hyperphagia in a subject in need thereof using desacyl ghrelin or an active fragment thereof.

In one embodiment of the treatment, the desacyl ghrelin comprises a peptide having an amino acid sequence according to any of SEQ ID NO:1 to SEQ ID NO:21.

In one embodiment of the treatment, the active fragment of the desacyl ghrelin comprises a peptide having an amino acid sequence according to any of SEQ ID NO:22 to SEQ ID NO:25.

Amino acid sequences of desacyl ghrelin as considered herein are as follows (Kojima and Kangawa 2005): (a) Mammalian

| | | |
|---|---|---|
| Human | GSSFLSPEHQRVQQRKESKKPPAKLQPR | SEQ ID NO:1 |
| Rhesus monkey | GSSFLSPEHQRAQQRKESKKPPAKLQPR | SEQ ID NO:2 |
| Mouse | GSSFLSPEHQKAQQRKESKKPPAKLQPR | SEQ ID NO:3 |
| Mongolian gerbil | GSSFLSPEHQKTQQRKESKKPPAKLQPR | SEQ ID NO:4 |
| Rat | GSSFLSPEHQKAQQRKESKKPPAKLQPR | SEQ ID NO:5 |
| Dog | GSSFLSPEHQKLQQRKESKKPPAKLQPR | SEQ ID NO:6 |
| Porcine | GSSFLSPEHQKVQQRKESKKPAAKLKPR | SEQ ID NO:7 |
| Sheep | GSSFLSPEHQKLQ-RKEPKKPSGRLKPR | SEQ ID NO:8 |
| Bovine | GSSFLSPEHQKLQ-RKEAKKPSGRLKPR | SEQ ID NO:9 |

(b) Avian

|         |                               |               |
|---------|-------------------------------|---------------|
| Chicken | GSSFLSPTYKNIQQQKDTRKPTARLH     | SEQ ID NO:10  |
| Duck    | GSSFLSPEFKKIQQQNDPTKTTAKIH     | SEQ ID NO:11  |
| Emu     | GSSFLSPDYKKIQQRKDPRKPTTKLH     | SEQ ID NO:12  |
| Goose   | GSSFLSPEFKKIQQQNDPAKATAKIH     | SEQ ID NO:13  |
| Turkey  | GSSFLSPAYKNIQQQKDTRKPTARLHPR   | SEQ ID NO:14  |

(c) Fish

|                 |                              |               |
|-----------------|------------------------------|---------------|
| Rainbow trout 1 | GSSFLSPSQKPQVRQGKGK-PPRV-amide | SEQ ID NO:15 |
| Rainbow trout 2 | GSSFLSPSQKPQGKGK-PPRV-amide   | SEQ ID NO:16  |
| Japanese eel    | GSSFLSPSQRPQGKDKKPPRV-amide   | SEQ ID NO:17  |
| Goldfish        | GTSFLSPAQKPQ--GRRPPRM-amide   | SEQ ID NO:18  |
| Zebrafish       | GTSFLSPTQKPQ--GRRPPRV-amide   | SEQ ID NO:19  |
| Tilapia         | GSSFLSPSQKPQNKVK-SSRI-amide   | SEQ ID NO:20  |

(d) Amphibian

|          |                               |              |
|----------|-------------------------------|--------------|
| Bullfrog | GLTFLSPADMQKIAERQSQNKLRHGNMN  | SEQ ID NO:21 |

Amino acid sequences of desacyl ghrelin and desacyl ghrelin fragments as considered herein are distributed by Peptides International, Inc:

des-*n*-octanoyl-[Ser3]-ghrelin (human)

Gly-Ser-Ser-Phe-Leu-Ser-Pro-Glu-His-Gln-Arg-Val-Gln-Gln-Arg-Lys-Glu-Ser-Lys-Lys-

Pro-Pro-Ala-Lys-Leu-Gln-Pro-Arg

GSSFLSPEHQRVQQRKESKKPPAKLQPR          SEQ ID NO:1

des-*n*-octanoyl-[Ser3]-ghrelin (rat)

Gly-Ser-Ser-Phe-Leu-Ser-Pro-Glu-His-Gln-Lys-Ala-Gln-Gln-Arg-Lys-Glu-Ser-Lys-Lys-

Pro-Pro-Ala-Lys-Leu-Gln-Pro-Arg

GSSFLSPEHQKAQQRKESKKPPAKLQPR          SEQ ID NO:5

des-n-octanoyl-[Ser$^3$]-ghrelin (human, 1-18)

H-Gly-Ser-Ser-Phe-Leu-Ser-Pro-Glu-His-Gln-Arg-Val-Gln-Gln-Arg-Lys-Glu-Ser-NH$_2$

GSSFLSPEHQRVQQRKES                    SEQ ID NO:22

des-n-octanoyl-[Ser³]-ghrelin (human, 1-14)

H-Gly-Ser-Ser-Phe-Leu-Ser-Pro-Glu-His-Gln-Arg-Val-Gln-Gln-OH

GSSFLSPEHQRVQQ                                                    SEQ ID NO:23


des-*n*-octanoyl-[Ser³]-ghrelin (human, rat, 1-10)

H-Gly-Ser-Ser-Phe-Leu-Ser-Pro-Glu-His-Gln-NH$_2$

GSSFLSPEHQ                                                        SEQ ID NO:24


des-*n*-octanoyl-[Ser³]-ghrelin (human, rat, 1-5)

H-Gly-Ser-Ser-Phe-Leu-NH$_2$

GSSFL                                                            SEQ ID NO:25

The term "hyperphagia" means an excessive ingestion of food beyond that needed for basic energy requirements. Hyperphagic conditions may occur in association with the central nervous system (CNS) and its disorders. Hyperphagia may occur in psychiatric disorders such as depression, anxiety and schizophrenia. A subgroup of patients with anxiety overeat and gain weight as do some patients with unipolar depression and in the depressive phase of a bipolar disorder. Hyperphagia may be associated with impaired satiety.

The term "eating disorder" is a complex compulsion to eat, or not eat, in a way which disturbs physical and mental health. One of the most widely and rapidly spreading eating disorder is compulsive overeating or "binge eating disorder" (BED). Binge eating disorder is a psychiatric disorder in which a subject shows the following symptoms: periodically does not exercise control over consumption of food; eats an unusually large amount of food at one time - more than a normal person would eat in the same amount of time; eats much more quickly during binge episodes than during normal eating episodes; eats until physically uncomfortable; eats large amounts of food even when they are not really hungry; usually eats alone during binge eating episodes, in order to avoid discovery of the disorder; often eats alone during periods of normal eating, owing to feelings of embarrassment about food; feels disgusted, depressed, or guilty after binge eating. Most people with this problem are either overweight or obese. Eating disorders may cause severe immediate and long-term health issues and can cause death.

The term "central nervous disorder" means a neurologic disorder, in particular a psychiatric disorder such as binge eating disorder.

The term "an adult" means a sexually mature subject.

The "body-mass index" BMI is calculated according to the following formula:

$$BMI = \text{body weight [kg]}/(\text{body height [m]})^2$$

The aim of the study underlying the present invention was to investigate whether desacyl ghrelin affects food intake, body weight gain and Fos expression pattern in the PVN, in the ARC, in the nucleus tractus solitarius (NTS), and in the area postrema (AP) of the brainstem in adult obese Zucker-rats. Zucker-rats (fa/fa) are a genetic model of obesity in rats caused by a missense-mutation of the leptin-receptor. A further aim was to investigate the interaction between ghrelin and desacyl ghrelin administered ip in the modulation of food intake in freely fed rats.

In the study, we demonstrated that ip injection of 5 nmol desacyl ghrelin/rat induces a significant decrease in food intake in Zucker-rats while 1 nmol/rat does not have any effect on feeding behaviour. Furthermore, chronic administration of desacyl ghrelin for a period of eight days leads to a significant stagnation of body weight gain which was not compensated in the following four days after treatment. Moreover, peripheral injection of 5 nmol desacyl ghrelin/rat caused a significant increase in neuronal activity in hypothalamic ARC and PVN whereas a decrease of Fos-expression was shown in NTS.

Further data show that the orexigenic effect induced by peripheral ghrelin is inhibited by desacyl ghrelin administered ip simultaneously at similar or higher doses in freely fed rats suggesting that desacyl ghrelin may be involved in the regulation of energy homeostasis in rodents, at least partially by counteracting the orexigenic effect of ghrelin.

In conclusion, our findings show that ip injection of desacyl ghrelin modulates food intake and body weight gain in adult obese Zucker-rats. Further, desacyl ghrelin injected ip increased Fos expression in the PVN and ARC, and decreased Fos expression in the NTS. These results suggest that peripheral desacyl ghrelin acts via central pathways to regulate energy homoeostasis in Zucker-rats.

The finding, that desacyl ghrelin modulates body weight gain in addition to food intake was surprising. Previous studies on the effect of desacyl ghrelin on food intake and body weight gain were conducted with normal weight mice and rats only (Asakawa et al. 2005; Chen et al. 2005a; Neary et al. 2006; Toshinai et al. 2006). However, these results can not be easily transferred to situations where the regulation of food intake is impaired, e.g. eating disorders. Moreover, an agent reducing food intake must not necessarily affect the body weight. For example, a single administration of CCK reduces food intake in laboratory animals, but continued administration does not result in body weight loss. Although the amount of food intake per meal is indeed reduced by CCK, a compensation by more frequent food intake is observed (Crawley and Beinfeld 1983; West et al. 1984). Also the injection of leptin induces a reduction of food intake, but does not result in body weight loss after chronic administration (Halaas et al. 1997).

**Detailed description of the invention**

The present invention shall now be described in more detail on the basis of the following examples and by making references to the table and to the attached figures.

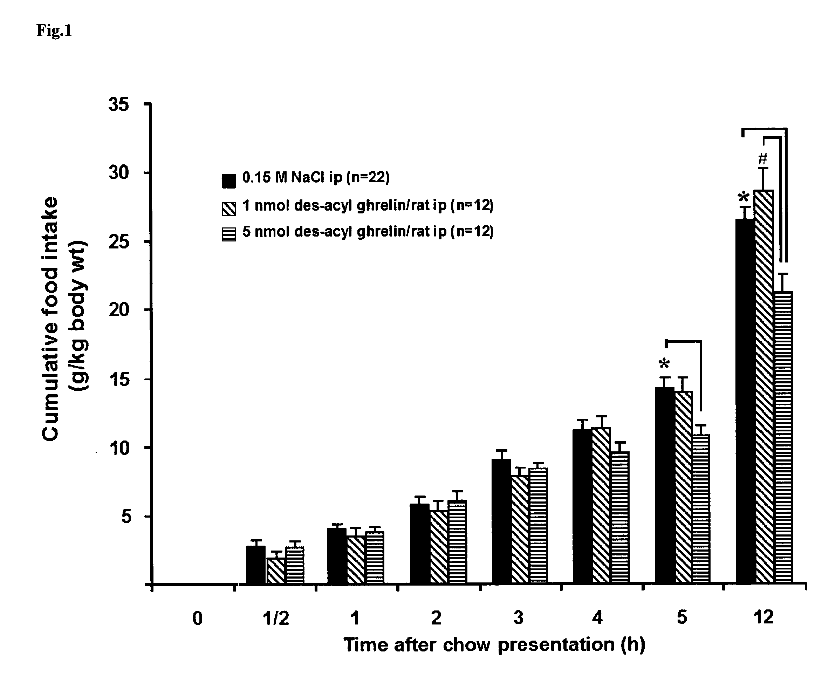

Figure 1     shows the effect of desacyl ghrelin on food intake at the dark phase under *ad libitum* feeding conditions. Zucker-rats were injected ip with 1 or 5 nmol desacyl ghrelin/rat or with 0.15 M NaCl solution (vehicle) 15 minutes before the beginning of the darkphase. 5 nmol desacyl ghrelin decreased cumulative food intake after five hours at the dark phase; whereby, this effect was statistically significant for 5 and 12 hours. 1 nmol desacyl ghrelin had no suppressive effect on food intake during the whole observation period. The data are expressed as mean and SEM. *$p < 0.05$ *vs.* vehicle. #$p < 0.05$ *vs.* 1 nmol desacyl ghrelin.

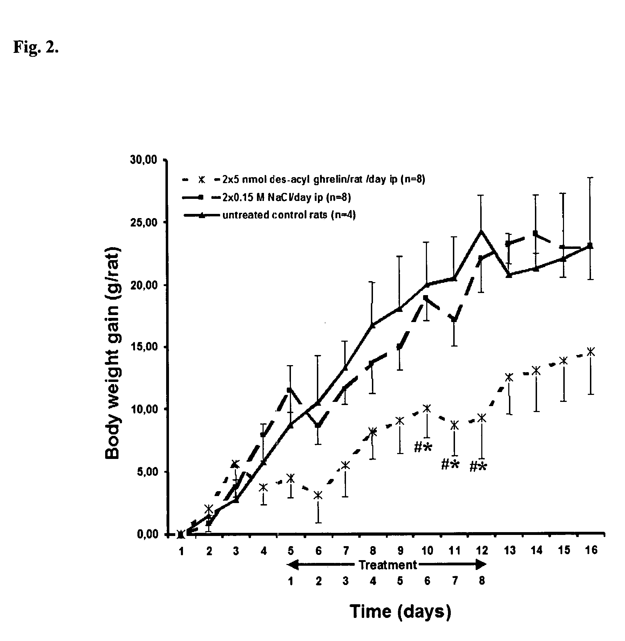

Figure 2     shows the effect of peripheral desacyl ghrelin on body weight gain after ip injection.
Injection of 5 nmol desacyl ghrelin twice a day for eight days led to a significant stagnation of body weight gain from day six of treatment compared to un-treated controls. This plateau in the development of body weight gain continued until the end of treatment. Afterwards body weight gain of the desacyl ghrelin treated rats developed similar to vehicle group, so there was no compensation of the difference in body weight gain induced by desacyl ghrelin treatment. The data are expressed as mean and SEM. *$p < 0.05$ *vs.* vehicle, #$p < 0.05$ *vs.* untreated control rats.

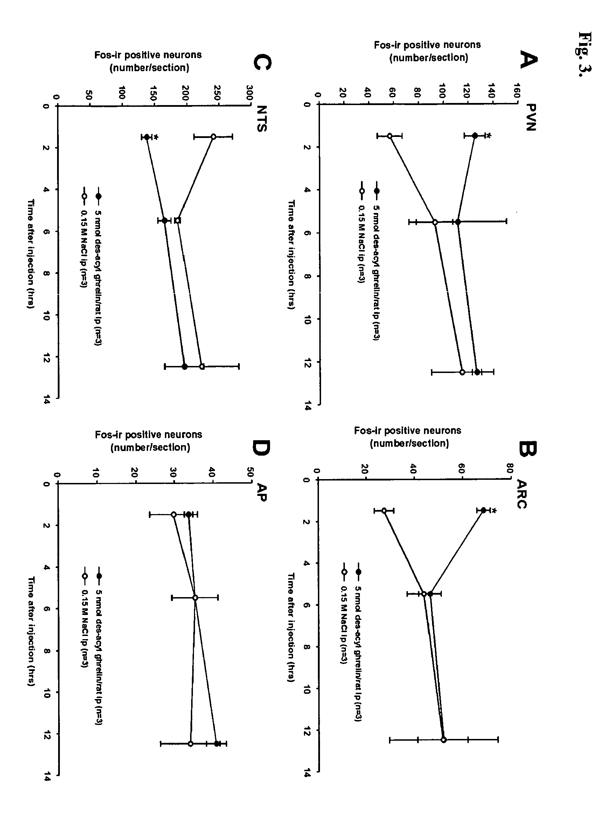

Figure 3     shows the effects of peripheral desacyl ghrelin on Fos-immunoreactivity (Fosir) in the hypothalamus and brainstem.
90 min after ip injection 5 nmol desacyl ghrelin/rat induced significantly an increase in the density of Fos positive neurons in the paraventricular nucleus (PVN) and arcuate nucleus (ARC) compared to vehicle treated Zucker-rats.
Desacyl ghrelin decreased neuronal activity in the nucleus tractus solitarius (NTS) after 90 min post injection. 4.5 and 12.5 h after ip injection no significant effect on Fos expression pattern in the PVN, ARC and NTS was observed. Desacyl ghrelin had no effect on neuronal activity in the AP over the complete observation period. The data are expressed as mean and SEM. *$p < 0.05$.

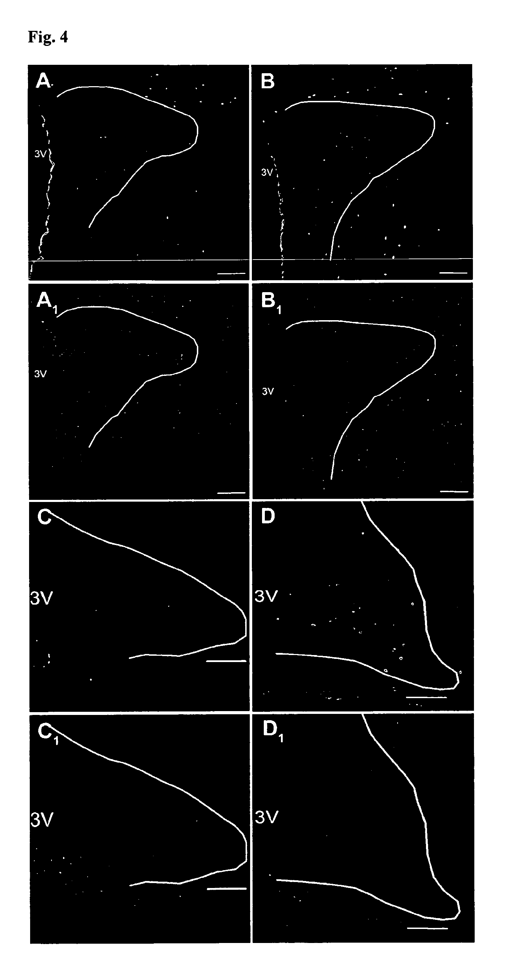

Figure 4     shows the effects of peripheral desacyl ghrelin on Fos-ir in the PVN and in the ARC of the hypothalamus. 5 nmol desacyl ghrelin/rat injected ip induces Fos-ir in the PVN and ARC in fed rats. Neurons in the PVN (B) and ARC (D) showed Fos-ir (green staining) 90 min after injection of the peptide while after saline injection (A and C) only few Fos-ir positive neurons were observed. Cell nuclei are stained red as a result of the counterstaining with propidium iodide in the same slice of desacyl ghrelin- ($B_1$ and $D_1$) and saline-treated ($A_1$ and $C_1$) rats. The white outer line delineates the area of the PVN. The white scale bar represents 100 $\mu$m. 3V = third ventricle.

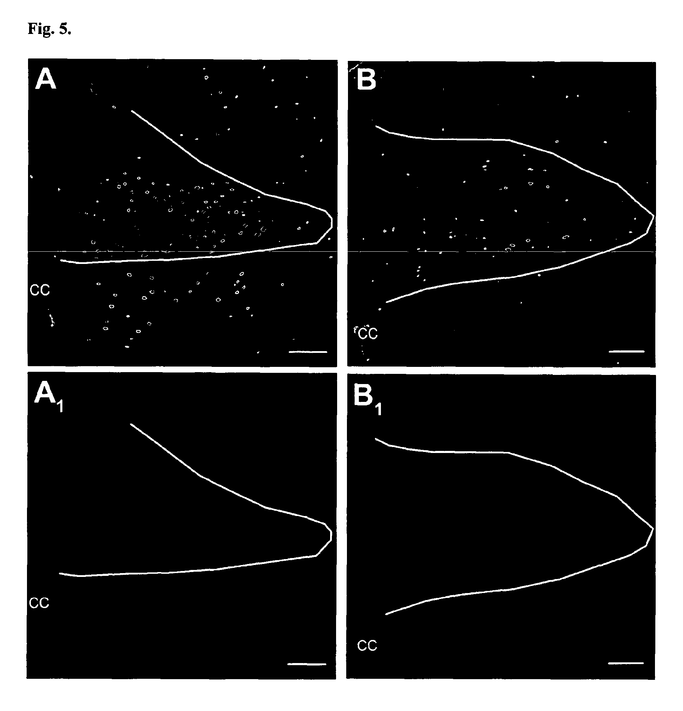

Figure 5    shows the effects of peripheral desacyl ghrelin on Fos-ir in the NTS and in the AP of the brainstem. 5 nmol desacyl ghrelin/rat (B) decreased neuronal activity (green staining) in the NTS after 90 min post injection compared to the NaCl (A) treated Zuckerrats. Cell nuclei are stained red as a result of the counterstaining with propidium iodide in the same slice of desacyl ghrelin- ($B_1$) and saline-treated ($A_1$) rats. The white outer line delineates the area of the NTS. The white scale bar represents 100 $\mu$m. cc = central canal.

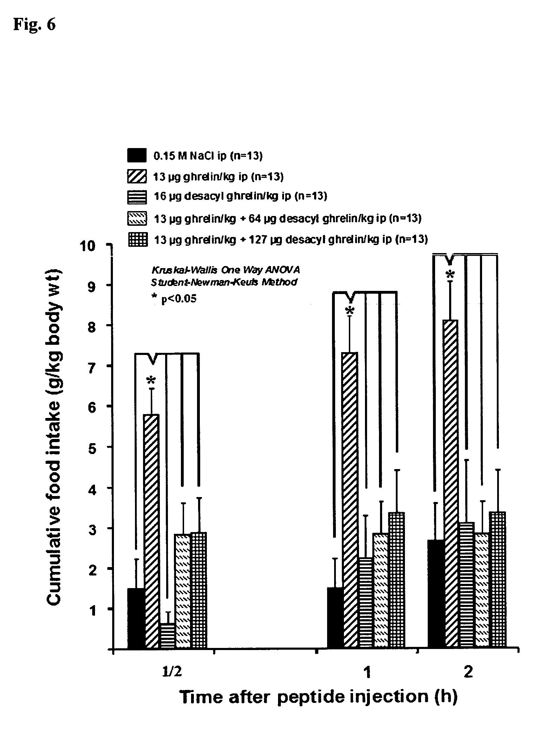

Figure 6    shows the effects of ghrelin and desacyl ghrelin injected alone or simultaneously ip on 2 h-food intake. Freely fed rats were injected ip with vehicle, desacyl ghrelin (64 $\mu$g/kg), ghrelin (13 $\mu$g/kg) plus desacyl ghrelin (64 or 127 $\mu$g/kg), or ghrelin (13 $\mu$g/kg), and cumulative food intake (expressed as g/kg body weight) was measured at 0.5, 1 and 2 h. Data are expressed as mean and SEM of number of rats indicated in parenthesis. *$p < 0.05$.

EXAMPLES

EXAMPLE 1: *Materials and Methods*

*1. Animals*

*1.1. Studies underlying Figs. I to 5*

Male adult Zucker rats (Harlan-Winkelmann Co., Borchen, Germany) weighting 650 g were housed in groups of 3 rats/cage under conditions of controlled illumination (12:12 h light/dark cycle, lights on/off: 6:30 a.m./6:30 p.m.), humidity, and temperature (22 $\pm$ 2°C). Animals were fed with a standard rat diet (Altromin®, Lage, Germany) and tap water *ad libitum.* All animals were trained daily to be accustomed to the experimental conditions for 14 days before starting the experiments. During the handling phase, the back position was practiced to make animals familiar with receiving an intraperitoneal (ip) injection. Animals used for neuronal mapping (Fos-ir) were also handled daily for 14 days before the start of the experiment and similarly trained to be held in the back position for ip injection.
Animal care and experimental procedures followed institutional ethic guidelines and conformed to the requirements of the state authority for animal research conduct.

*1.2. Study underlying Fig. 6*

Male adult SD rats (Harlan-Winkelmann Co., Borchen, Germany) weighting 250 g were housed in groups of 4 rats/cage under conditions of controlled illumination (12:12 h light/dark cycle, lights on/off: 6:30 a.m./6:30 p.m.), humidity, and temperature (22 $\pm$ 2°C). Animals were fed with a standard rat diet (Altromin®, Lage, Germany) and tap water *ad libitum.* All animals were trained daily to be accustomed to the experimental conditions for 14 days before starting the experiments. During the handling phase, the back position was practiced to make animals familiar with receiving an ip injection.
Animal care and experimental procedures followed institutional ethic guidelines and conformed to the requirements of the state authority for animal research conduct.

*2. Peptide preparation*

*2.1. Studies underlying Figs. I to 5*

Rat desacyl ghrelin (1 mg/ml; Peptides International, Louisville, USA) was dissolved in distilled water and stored at -20°C. Immediately before starting the experiments, peptides were diluted in vehicle solution consisting of sterile 0.15 M NaCl (Braun, Melsungen, Germany) to reach the final concentration of 1 and 5 nmol desacyl ghrelin.

*2.2. Study underlying Fig. 6*

Rat desacyl ghrelin (0.5 mg/ml; Peptides International, Louisville, USA) or rat ghrelin (1 mg/ml) was dissolved in distilled water and stored at -20°C. Immediately before starting the experiments, peptides were diluted in vehicle solution consisting of sterile 0.15 M NaCl (Braun, Melsungen, Germany) to reach the final concentration of 13 $\mu$g (~4 nmol) ghrelin/kg, and 64 $\mu$g (~20 nmol) or 127 $\mu$g (~40 nmol) desacyl ghrelin/kg body weight. Peptide solutions were kept on ice for the duration of the experiments. Doses of peptides were selected based on previous studies *(Chen et al.* 2005a; Kobelt *et al.* 2005; *Toshinai et al.* 2006).

*3. Experimental protocols*

*3.1. Study I*

Study 1 refers to the effects of peripheral desacyl ghrelin on food intake injected ip at the beginning of the dark phase. The cumulative food intake was measured over a period of 12 h post ip injection of 1 and 5 nmol desacyl ghrelin/rat or 0.15 M NaCl during dark phase.

In more detail, on the day of the experiment, randomized rats were injected ip (final volume: 500 $\mu$l) with vehicle solution (0.15 M NaCl, n=22) or 1 (n=12) and 5 (n=12) nmol desacyl ghrelin/rat 15 minutes before the dark phase started, and returned to single housing cages immediately after the injection. Five minutes before the dark phase started, pre-weighed rat chow was made available to the animals. Food intake was determined by measuring the difference between the preweighed standard chow and the weight of chow at the end of the first 30 min, 1, 2, 3, 4, 5, and 12 h after food exposure.

*3.2. Study 2*

Study 2 refers to the effect of peripheral desacyl ghrelin on body weight gain after ip injection. Body weight gain was monitored over a period of 16 days with daily ip injection of 2 x 5 nmol desacyl ghrelin/rat for eight days.

In more detail, the effect of daily ip injected desacyl ghrelin (5 nmol /rat) on body weight gain was investigated in adult obese Zucker-rats. Rats were divided in three groups: Two of them (n=8/group) were treated ip with 0.15 M NaCl solution or 5 nmol desacyl ghrelin/rat, respectively. Another untreated control group (n=4) served as an indicator for stress-induced changes in body weight gain.

Animals were weighted daily at 6:30 A.M. in a period of four days before the treating started, during the eight days of application and four days afterwards. 5 nmol desacyl ghrelin/rat or vehicle in a volume of 500 $\mu$l was injected ip at the beginning of both, light- and darkphase for eight days. Progression of body weight was acquired as cumulative body weight gain (in g) over the entire 16 days.

*3.3. Study 3*

Study 3 refers to the effects of peripheral desacyl ghrelin on Fos-immunoreactivity in the hypothalamus and brain stem. The Fos expression pattern during dark phase after ip injection of 5 nmol desacyl ghrelin/rat was investigated.

In more detail, freely fed rats (n=3/group) were injected ip (final volume 0.5 ml) with vehicle (0.15 M NaCl) or desacyl ghrelin (5 nmol/rat) immediately before the dark phase started. Immediately after the injection animals had *ad libitum* access to food and water. At 1.5 5.5 and 12.5 h after ip injection, animals were deeply anesthetized with 100 mg/kg body weight ketamine (Ketanest®, Curamed, Karlsruhe, Germany) and 10 mg/kg body weight xylazine (Rompun® 2%, Bayer, Leverkusen, Germany), and heparinized with 2.500 U heparin (Liquemin®, Hoffmann-La Roche, Grenzach-Whylen, Germany). Transcardial perfusion was performed as described before (Geisler *et al.* 2002). It started with a 10-s flush of a plasma substitute (Longasteril® 70, Fresenius, Bad Homburg, Germany), followed by a mixture of 4% w/v paraformaldehyde, 0.05% v/v glutaraldehyde, and 0.2% v/v picric acid in 0.1 M phosphate buffer, pH 7.4, for 30 min and finished with a 5% w/v sucrose solution for 5 min. After dissection, brains were kept in a 5% w/v sucrose solution overnight and then cut into 1.0 to 4.5 mm coronal blocks enclosing the hypothalamic and brainstem regions repectively using a plexiglass brain matrix. For cryoprotection, blocks were moved through a sucrose gradient (15% w/v and 27.3% w/v), then shock-frozen in hexane at -70°C, and stored at -80°C until further processing.

*3.4. Study underlying Fig. 6*

The experiments were performed at 2.5 h after the start of the light cycle in freely fed rats. At that time period, freely fed rats have their lowest food intake.

On the day of the experiment, freely fed animals were injected ip simultaneously (final volume: 0.5 ml) with vehicle plus vehicle (0.15 M NaCl + 0.15 M NaCl, n=13), ghrelin plus vehicle (13 $\mu$g/kg + 0.15 M NaCl, n=13), ghrelin (13 $\mu$g/kg) plus 64 $\mu$g/kg or 127 $\mu$g/kg desacyl ghrelin (n =13 in both groups), or vehicle plus 64 $\mu$g/kg desacyl ghrelin (n=13) and returned to single housing cages immediately after the injection. Thereafter, preweighed rat chow was made available to the animals. Food intake was determined by measuring the difference between the preweighed standard chow and the weight of chow at the end of the first 30 min, 1 h and 2 h food exposure.

*4. Immunohistochemistry: Staining for Fos-immunoreactivity (Fos-ir)*

First, 25 $\mu$m free-floating sections were pretreated with 1% w/v sodium borohydride (in phosphate buffered saline; PBS) for 15 min. Subsequently, sections were incubated in a solution containing 5% w/v bovine serum albumin (BSA) and 0.3% v/v Triton X-100 in PBS for 60 min for blockade of unspecific antibody binding. Thereafter, the diluted primary antibody solution (rabbit anti-rat c-Fos protein; Oncogene Research Products, Boston, USA; 1:4.000 in a solution of 5% w/v BSA, 0.3% v/v Triton X-100, and 0.1% w/v sodium azide in PBS) was applied for 48 h at room temperature.

After rinsing sections in PBS three times and incubation in a solution containing 5% w/v BSA and 0.3% v/v Triton X-100 for 60 min, FITC-labelled goat-anti-rabbit IgG (Sigma, St. Louis, USA) was applied for 12 h at room temperature in an appropriate dilution (1:600 in 5% w/v BSA in PBS). Sections were rinsed in PBS three times again and stained with propidium iodide (2.5 $\mu$g/ml in PBS) for 15 min to counterstain cell chromatin. Tissue sections were finally embedded in 15 $\mu$l anti-fading solution (100 mg/ml 1,4-diazabicyclo[2.2.2]octan (Sigma, St. Louis, USA) in 90 % v/v glycerin, 10 % v/v PBS, pH 7.4), and analyzed using a confocal laser scanning microscope (cLSM 510 Meta, Carl Zeiss, Germany).

*5. Data and statistical analysis*

*5.1. Effects of desacyl ghrelin ip on food intake*

The cumulative food intake monitored at the end of the first 30 min, 1, 2, 3, 4, 5 and 12 h after peptide or vehicle injection was expressed as food intake (g)/body weight (kg) (Ruter *et al.* 2003). Data were expressed as mean $\pm$ SEM and analyzed by ANOVA. Differences between groups were evaluated by the Least Significant Difference test; p<0.05 was considered significant.

*5.2. Effects of desacyl ghrelin ip on body weight gain*

The cumulative body weight gain in the experiment with the daily ip administration of 2x5 nmol desacyl ghrelin/rat monitored over a time period of 16 days was expressed as body weight in g. Data were expressed as mean $\pm$ SEM, and differences between the groups were evaluated by the Least Significant Difference (LSD) test; p<0.05 was considered significant.

*5.3. Effects of desacyl ghrelin ip on Fos-immunoreactivity*

Semi-quantitative assessment of Fos-immunoreactivity (Fos-ir) was achieved by counting the number of Fos-ir positive neurons as described before *(Kobelt et al.* 2005). Neurons with green nuclear staining were considered Fos-ir-positive. Every second of all consecutive coronal 25 $\mu$m sections was counted bilaterally for Fos-ir positive staining in the PVN, ARC, NTS and unilateral in the AP throughout their rostrocaudal extent. Fos positive neurons were counted in 10 sections per rat of the PVN, and 15 sections per rat of the ARC, NTS and AP. Anatomic correlations were made according to landmarks given in Paxinos and Watson's stereotaxic atlas (Paxinos and Watson 1997). The investigator counting the number of Fos-ir positive cells was blinded to treatments received by the animals.

The average number of Fos-ir positive neurons per slide for the brain nuclei mentioned above was calculated for each rat. All data were expressed as means $\pm$ SEM and analyzed by 2-way ANOVA. Differences between groups were evaluated by the LSD post hoc test with p<0.05 was considered significant.

*5.4. Effects of co-adminisiration of desacyl ghrelin and ghrelin*

The cumulative food intake monitored at the end of the first 30 min, 1 h, and 2 h after peptide or vehicle injection was expressed as food intake (g)/body weight (kg) (Ruter *et al.* 2003). Data were expressed as mean $\pm$ SEM and analyzed by Kruskal-Wallis One Way ANOVA. Differences between groups were evaluated by the Student-Newman-Keuls method; p < 0.05 was considered significant.

EXAMPLE 2: *Results of Study I*

Rat desacyl ghrelin at a dose of 5 nmol/rat ip significantly decreased food intake after five hours post injection compared to the vehicle group (mean $\pm$ SEM: 10.79 $\pm$ 0.74 g/kg body weight *vs.* 14.18 $\pm$ 0.88 g/kg body weight, p<0.016; Fig. 1). The cumulative food intake was still significantly decreased after 12 h post injection in the group treated with 5 nmol desacyl ghrelin (21.21$\pm$ 1.33 g/kg body weight; Fig. 1) compared to the vehicle (26.45 $\pm$ 0.99 g/kg body weight, p<0.005; Fig. 1) and 1 nmol desacyl ghrelin group (28.58 $\pm$ 1.61 g/kg body weight, p<0.001; Fig. 1). No effect on cumulative food intake during the entire observation period was observed in animals treated with 1 nmol desacyl ghrelin ip.

EP 2 067 481 A1

EXAMPLE 3 : *Results of Study 2*

Compared to the untreated control group, body weight gain was not influenced by administration of vehicle (Fig. 2). In contrast, injection of 5 nmol desacyl ghrelin twice a day for eight days led to a significant stagnation of body weight gain from day six of treatment compared to vehicle and to the untreated control animals (Fig. 2 and Table 1).

This plateau in the development of body weight continued until the end of treatment. Afterwards body weight gain of the desacyl ghrelin treated animals developed similar to vehicle group, so there was no compensation of the difference in body weight gain induced by desacyl ghrelin treatment.

Table 1: Effect of peripheral desacyl ghrelin on body weight gain after ip injection

| day of treatment | desacyl ghrelin (g/rat) | vehicle (g/rat) | vehicle *vs.* desacyl ghrelin | untreated control rats (g/rat) | untreated control *vs.* desacyl ghrelin |
|---|---|---|---|---|---|
| 6 | $10 \pm 2.35$ | $18.88 \pm 1.81$ | 0.009 | $20 \pm 3.34$ | 0.016 |
| 7 | $8.63 \pm 2.42$ | $17.15 \pm 2.16$ | 0.018 | $20.50 \pm 3.28$ | 0.008 |
| 8 | $9.25 \pm 3.29$ | $22 \pm 2.69$ | 0.006 | $24.25 \pm 2.84$ | 0.007 |
| (The data are expressed as mean and SEM) | | | | | |

Our results obtained in Study 1 and Study 2 confirm the anorexinogenic effect of desacyl ghrelin observed in lean rats and mice (Asakawa *et al.* 2005). In addition, there is congruence with findings in transgenic mice overexpressing desacyl ghrelin which exhibit a reduced food intake and a lower body weight than wildtype littermates *(Asakawa et al.* 2005). Our investigation was performed during dark phase, because satiety signals develop the strongest effect at this time, which is the period of the highest physiological food intake. This observation was confirmed by other studies: Icv and ip application of desacyl ghrelin (5 nmol) significantly suppressed food intake in 16 h food deprived animals during light phase. In contrast, in *ad libitum* fed rats there was only a tendency towards a reduction (Asakawa *et al.* 2005) or no influence on food intake (Chen *et al.* 2005a). During the dark phase both fasted and *ad libitum* fed rats showed a significant inhibitory effect of desacyl ghelin on feeding behaviour (Chen *et al.* 2005a). Recently, Neary *et al.* (2006) did not find any effect of desacyl ghrelin on feeding behaviour in 20 h fasted or freely fed mice. Contrary, Toshinai *et al.* (2006) found that icv injection of desacyl ghrelin during light phase induces an increase of food intake in unfasted rats and mice whilst in fasted animals there is no change in feeding behaviour after icv or ip administration. These results were reproducible in GHS, but not in orexin-deficient mice (Toshinai *et al.* 2006). However, these findings suggest that desacyl ghrelin displays its effects via interaction with orexin-expressing neurons independent from GHS-receptor (Toshinai *et al.* 2006).

There are further differences in the results concerning the latency between injection and effect: Whilst the significant reduction of food intake in 16 h fasted mice occured already 20 and 60 min after administration (Asakawa *et al.* 2005), in our study the anorexinogenic effect in *ad libitum* fed Zucker-rats reached statisticals significance 5 and 12 h post injection.

In our study chronic ip injection of desacyl ghrelin for eight days induced a significant stagnation of body weight gain, which had not been compensated in the following four days after treatment. Similar findings were found in transgenic mice, which over-express prepro-ghrelin what leads to 30-fold increased plasma levels of desacyl ghrelin but normal ghrelin synthesis (Asakawa *et al.* 2005). These animals showed a significant reduction of body weight, a decrease of body fat mass and they tend to a smaller body length (Asakawa *et al.* 2005). Moreover, in this study a significant reduction of food intake and an inhibitory effect on gastric emptying could be demonstrated (Asakawa *et al.* 2005). Previous investigations showed that in the same way peripherally administrated desacyl ghrelin suppressed feeding behaviour in mice and inhibited gastric emptying (Asakawa *et al.* 2005). According to these observations the slower gastric emptying could lead to an augmented distension of the stomach. After reaching the maximum accomodation, vagal afferences induce an enduring satiety (Geliebter 1988). In this context, it is remarkable that the gastric motility is influenced isochronic by either ip or ivc application whilst the inhibitory effect on food intake reaches statistical significance 20 min after ip injection but 60 min after icv administration (Asakawa *et al.* 2005).

EXAMPLE 4: *Results of Study 3*

90 min after ip injection of 5 nmol desacyl ghrelin/rat induced a robust increase in the density of Fos positive neurons in the PVN (mean $\pm$ SEM: $125.53 \pm 8.48$ *vs.* $56.84 \pm 10.02$, p<0.001; Fig 3A and 4) and ARC ($68.63 \pm 2.77$ *vs.* $27.33 \pm 4.05$, p = 0.02; Fig 3B and 4) compared to vehicle treated animals. Interestingly, in contrast to the ARC and PVN ip injection of desacyl ghrelin decreased neuronal activity in the NTS after 90 min post injection compared to rats treated with 0.15 M NaCl solution (137.73 $\pm$ 8.15 *vs.* 241.39 $\pm$ 30.19, p < 0.001; Fig 3C and 5).

4.5 and 12.5 h after ip injection no significant effect on Fos expression pattern in the PVN, ARC and NTS was observed (Figs. 3A, B, and C). However, desacyl ghrelin at a dose of 5 nmol had no effect on neuronal activity in the AP over the complete observation period (Fig 3D), as assessed by Fos-ir.

The results of the present study concerning the Fos expression pattern after desacyl ghrelin administration extensively correspond to findings in lean mice. In previous studies, ip injection of desacyl ghrelin induced a significant increase of For-ir positive neurons in the parvocellular part of the PVN, and the ventrolateral ARC (Asakawa *et al.* 2005; Chen *et al.* 2005a). In the PVN some desacyl ghrelin activated neurons expressed the neuropeptide CRF (Chen *et al.* 2005a). In contrast, Toshinai *et al.* (2006) did not find any changes in Fos-expression neither in the PNV nor in ARC after icv application of desacyl ghrelin. Modulation of neuronal activity in the NTS was not observed in these studies (Asakawa *et al.* 2005; Chen *et al.* 2005a). In our study we used Zucker-rats, which possess a missense mutation in leptin receptor gene and exhibit a malfunctioning satiety regulation. This causes enduring hyperphagia and consecutive obesity. As they had *ad libitum* access to food in the meantime between injection and perfusion, animals, especially of the vehicle group, continued eating great amounts. According to chemical and mechanical stimulation vagal afferences were activated by food intake and caused accelerated Fos-expression in the NTS. The decrease in Fos-expression after des-acyl ghrelin treatment seems to be due to the suppressed food intake but no effect mediated directly by desacyl ghrelin because the peptide is also effective after capsaicin-treatment and therefore independent of vagal afferences (Chen *et al.* 2005a). Unlike, the effect of intraperitoneally applied acylated ghrelin is completely abolished after capsaicin-treatment (Chen *et al.* 2005a). In contrast, total vagotomy also leads to annhilation of desacyl ghrelin induced inhibition of gastric motility. Hence, this effect seems to be controlled centrally and mediated by vagal efferences (Chen *et al.* 2005a). Thus, desacyl ghrelin receptors might be located in brainstem and hypothalamus rather than in peripheral tissues (Chen *et al.* 2005a).

It has been reported that desacyl ghrelin passed the blood-brain-barrier (Banks *et al.* 2002) and seems to act via central pathways mainly, whilst the orexinogenic effect of acylated ghrelin is mediated peripherely via vagal afferences (Chen *et al.* 2005a). The receptor involved in desacyl ghrelin actions is still unknown. But there are already findings about the further signal transduction: After intraperitoneal desacyl ghrelin administration a significant increase of anorexinogenic neuropeptides CART and urocortin was observed in the hypothalamus (Asakawa *et al.* 2005). Urocortin has a high selectivity for $CRF_2$-receptor (corticotropin releasing factor-receptor), whilst CRF has a strong affinity to the $CRF_1$-receptor (Chang *et al.* 1993; Coskun *et al.* 1997). CRF-receptor subtypes in the brain are involved in stress-related behaviour and physiological adaptation (Ando *et al.* 1998). The $CRF_1$-receptor mainly regulates anxiety (Takahashi 2001; Bale and Vale 2004), the $CRF_2$-receptor regulates feeding and gastrointestinal functions like gastric acid secretion and gastric emptying (Chen *et al.* 2002).

As another hint for the central effects of desacyl ghrelin might be considered that after icv administration the peptide's actions were observed after a dose of 1 nmol/rat whilst intraperitonaeal administration demanded 3 nmol/rat for significant decreased food intake (Asakawa *et al.* 2005). Interestingly the anorexinogenic effect occured faster after ip than icv administation (Asakawa *et al.* 2005).

EXAMPLE 5: *Results of the study underlying Fig. 6*

Ghrelin (13 $\mu$g /kg, ip) significantly increased food intake within the first half hour after ip injection compared to the vehicle plus vehicle group (5.76 $\pm$ 0.68 *vs.* 1.49 $\pm$ 0.72 g/kg body weight, p<0.05; Fig. 6). Desacyl ghrelin at both doses (64 and 127 $\mu$g/kg body weight) simultaneously injected with ghrelin (13 $\mu$g/kg body weight) abolished the stimulatory effect of ghrelin on food intake (2.81 $\pm$ 0.80 and 2.86 $\pm$ 0.87 g/kg, respectively; p<0.05; Fig. 6). Desacyl ghrelin at a dose of 64 $\mu$g/kg injected ip alone tended to decrease food intake during the first half hour compared to the vehicle plus vehicle group; although this did not reach statistical significance (0.61 $\pm$ 0.32 g/kg body weight, p>0.05; Fig. 6).

One hour after ghrelin administration, the significant increase in cumulative food intake compared to the vehicle plus vehicle group was still observed (7.29 $\pm$ 0.92 *vs* 1.49 $\pm$ 0.72 g/kg, p<0.05; Fig. 6). However, both doses of desacyl ghrelin (64 and 127 $\mu$g/kg) injected ip simultaneously with ghrelin (13 $\mu$g/kg) blocked the orexigenic effect of ghrelin on food intake (2.81 $\pm$ 0.80 g/kg body weight, p<0.05 and 3.34 $\pm$ 1.07 g/kg body weight, p<0.05, respectively; Fig. 6). Desacyl ghrelin injected ip alone at a dose of 64 $\mu$g/kg body weight in comparison to the vehicle plus vehicle group did not modify food intake at this time point (2.22 $\pm$ 1.06 g/kg body weight, p>0.05; Fig. 6).

At 2h after injection of ghrelin, the cumulative food intake in the ghrelin plus vehicle group was still significantly higher than in the vehicle plus vehicle group (7.71 $\pm$ 0.97 *vs.* 2.65 $\pm$ 0.93 g/kg body weight, p<0.05; Fig. 1). At this time point the inhibitory effect of desacyl ghrelin on ghrelin induced food intake at simultaneous injection of desacyl ghrelin (64 and 127 $\mu$g/kg body weight) and acyl ghrelin (13 $\mu$g/kg body weight) was maintained (2.81 $\pm$ 0.08 g/kg body weight, p<0.05 and 3.34 $\pm$ 1.07 g/kg body weight, p<0.05, respectively; Fig. 6). Desacyl ghrelin injected ip alone at a dose of 64 $\mu$g/kg body weight in comparison to the vehicle plus vehicle group did not have a modulating effect on food intake (3.09 $\pm$ 1.55 g/kg body weight, p>0.05; Fig. 6) at this time point.

In this study evidence is provided fort he first time for an inhibition of the stimulatory effect of peripheral ghrelin on food intake by peripheral desacyl ghrelin in rats. Ghrelin injected ip exerted an orexigenic effect in freely fed rats, which is in agreement with previous studies of our group and other groups (Kobelt *et al.* 2005; Nakazato *et al.* 2001; Toshinai *et al.* 2006).

Peripheral injection of ghrelin induces a significant increase of Fos positive neurons in the arcuate nucleus (ARC) in rats and mice (Hewson *et al.* 2000; Wang *et al.* 2002), and it has been found that Fos positive neurons in the ARC contain the orexigenic peptide neuropeptide Y (NPY) (Wang *et al.* 2002). Thus, one could speculate that NPY neurons in the ARC are involved in the mechanisms that regulate meal initiation. In the present study, both doses of desacyl ghrelin (64 and 127 μg/kg body weight ip) reduced the increase in food intake at 30 min after injection of ghrelin by about half resulting in a reduction of nutrients at both doses of about 50%. Thus, desacyl ghrelin acts to inhibit ghrelin induced food intake in rats in these experimental conditions. However, the underlying mechanisms still need to be elucidated.

Desacyl ghrelin has been shown to exert inhibitory effects on food intake in rodents (Chen *et al.* 2005a; 2005b) and transgenic mice over-expressing desacyl ghrelin displayed a decrease in food intake and body weight (Asakawa *et al.* 2005). Several research groups suggest a central action of desacyl ghrelin mainly because of its ability to cross the blood brain barrier due to the missing octanylation (Banks *et al.* 2002; Chen *et al.* 2005a). It has been shown that desacyl ghrelin-induced anorectic effects are not modulated by capsaicin treatment (Chen *et al.* 2002a). In contrast, while the vagal nerve has been shown to mediate acyl ghrelin's orexigenic effects this does not seem to be the case in transmission of anorectic desacyl ghrelin effects (Date *et al.* 2002; Chen *et al.* 2005a). However, a recent study reports that vagal afferents in mediating ghrelin effects on food intake are not necessary (Arnold *et al.* 2006). Furthermore, Fos expression in the nucleus tractus solitarii of the brainstem was not altered by desacyl ghrelin (Chen *et al.* 2005a).

Matsuda *et al.* (2006) recently examined the effects of ip as well as icv administration of desacyl ghrelin and ghrelin on food intake in goldfish (*Carassius αuratus*). They could show that both, ip and icv desacyl ghrelin injected prior to ghrelin blocked the orexigenic effect shown by ghrelin alone (Matsuda *et al.* 2006). Furthermore, pre-treatment with the neurotoxin capsaicin was able to prevent the inhibitory effects exerted by ghrelin on feeding but did not have a modulatory effect on the desacyl ghrelin related food intake (Matsuda *et al.* 2006). Single ip or icv applications of desacyl ghrelin did not modulate food intake in the goldfish (Matsuda *et al.* 2006). In the present study we found a similar effect; although a trend towards a reduced food intake in the rat could be observed during the first half hour.

A study by Gauna *et al.* (2007) revealed that administration of desacyl ghrelin dose-dependently enhanced the insulin response to an iv glucose load in rats predominantly in the portal vein (Gauna *et al.* 2007). This may lead to the supposition that desacyl ghrelin acts as a key player in the glucose/insulin homeostasis in the liver. Insulin, as an anorectic hormone, could be the mediator of desacyl ghrelin's opposing effects when co-injected with ghrelin. Ghrelin, on the other hand has been shown before to decrease insulin levels in humans (Gauna *et al.* 2004). Although, converse data exist concerning the interaction of acylated ghrelin with insulin and glucose (Broglio *et al.* 2001; Tassone *et al.* 2003; Vestergaard *et al.* 2007).

Another theory suggests that desacyl ghrelin exerts its inhibitory effects on food intake by modulation of gastrointestinal motor activity (Chen *et al.* 2005a). Further, Chen *et al.* 2005a) showed that acyl ghrelin was able to induce motor activity in rats in the fed state.

In contrast, desacyl ghrelin disrupted fasted motor activity in the gastric antrum but not in the duodenum in rats (Chen *et al.* 2005a). Urocortin, bombesin or CCK, all food inhibitory peptides, have also been shown to disrupt fasted motor activity in the gastrointestinal tract (Kihara *et al.* 2001; Poitras *et al.* 1983; Rodriguez-Membrilla *et al.* 1995). Thus, it might be possible that desacyl ghrelin exerts its actions via urocortins and/or POMC. It can be speculated that desacyl ghrelin impairs gastric motility which consequently blocks the orexigenic action of ghrelin in *ad libitum* fed rats via a strong sensation of fullness leading to ingestion of smaller amounts of food.

Other peptides have been shown to be capable of inhibiting ghrelin's orexigenic actions in rodents. We recently showed that CCK when co-administrated with ghrelin as well as co-injection of bombesin and ghrelin led to a reduction of the ghrelin induced food intake (Kobelt *et al.* 2005; 2006). In contrast, amylin was not able to modulate ghrelin induced food intake (Kobelt *et al.* 2006).

In conclusion, the present data show that the orexigenic effect induced by peripheral ghrelin is inhibited by desacyl ghrelin at both doses injected ip simultaneously with ghrelin in freely fed rats. These results suggest that desacyl ghrelin is involved in the regulation of energy homeostasis in rodents.

**References**

Ando T, Rivier J, Yanaihara H, Arimura A. Peripheral corticotropin-releasing factor mediates the elevation of plasma IL-6 by immobilization stress in rats. Am J Physiol 1998; 275:R1461-R1467.

Arnold M, Mura A, Langhans W, Geary N. Gut vagal afferents are not necessary for the eating-stimulatory effect of intraperitoneally injected ghrelin in the rat. J Neurosc 2006; 26:11052-11060.

Arvat E, Maccario M, di Vito L, Broglio F, Benso A, Gottero C, Papotti M, Muccioli G, Dieguez C, Casanueva FF, Deghenghi R, Camanni F, Ghigo E. Endocrine activities of ghrelin, a natural growth hormone secretagogue (GHS), in humans: comparison and interactions with hexarelin, a nonnatural peptidyl GHS, and GH-releasing hormone. J Clin Endocrinol Metab 2001; 86:1169-1174.

Asakawa A, Inui A, Kaga T, Yuzuriha H, Nagata T, Fujimiya M, Katsuura G, Makino S, Fujino MA, Kasuga M. A role of ghrelin in neuroendocrine and behavioral responses to stress in mice. Neuroendocrinology 2001 a; 74:143-147.

Asakawa A, Inui A, Kaga T, Yuzuriha H, Nagata T, Ueno N, Makino S, Fujimiya M, Niijima A, Fujino MA, Kasuga M. Ghrelin is an appetite-stimulatory signal from stomach with structural resemblance to motilin. Gastroenterology 2001b; 120:337-345.

Asakawa A, Inui A, Fujimiya M, Sakamaki R, Shinfuku N, Ueta Y, Meguid MM, Kasuga M. Stomach regulates energy balance via acylated ghrelin and desacyl ghrelin. Gut 2005;54:18-24.

Bale TL, Vale WW. CRF and CRF receptors: role in stress responsivity and other behaviors. Annu Rev Pharmacol Toxicol 2004; 44:525-557.

Banks WA, Tschop M, Robinson SM, Heiman ML. Extent and direction of ghrelin transport across the blood-brain barrier is determined by its unique primary structure. J Pharmacol Exp Ther 2002; 302:822-827.

Broglio F, Arvat E, Benso A, Gottero C, Muccioli G, Papotti M, van der Lely A. J. Deghenghi, R.; Ghigo, E. Ghrelin, a natural GH secretagogue produced by the stomach, induces hyperglycemia and reduces insulin secretion in humans. J. Clin. Endocrinol. Metab 2001; 86:5083-5086.

Chang CP, Pearse RV, O'Connell S, Rosenfeld MG. Identification of a seven transmembrane helix receptor for corticotropin-releasing factor and sauvagine in mammalian brain. Neuron 1993; 11:1187-1195.

Chen CY, Million M, Adelson DW, Martinez V, Rivier J, Tache Y. Intracisternal urocortin inhibits vagally stimulated gastric motility in rats: role of CRF(2). Br J Pharmacol 2002; 136:237-247.

Chen CY, Inui A, Asakawa A, Fujino K, Kato I, Chen CC, Ueno N, Fujimiya M. Des-acyl Ghrelin Acts by CRF Type 2 Receptors to Disrupt Fasted Stomach Motility in Conscious Rats. Gastroenterology 2005a; 129:8-25.

Chen CY, Chao Y, Chang FY, Chien EJ, Lee SD, Doong, ML. Intracisternal des-acyl ghrelin inhibits food intake and non-nutrient gastric emptying in conscious rats. Int J Mol Med 2005b; 16:695-699.

Coskun T, Bozkurt A, Alican I, Ozkutlu U, Kurtel H, Yegen BC. Pathways mediating CRF-induced inhibition of gastric emptying in rats. Regul Pept 1997; 69:113-120.

Crawley JN, Beinfeld MC. Rapid development of tolerance to the behavioural actions of cholecystokinin. Nature 1983; 302: 703-706

Date Y, Kojima M, Hosoda H, Sawaguchi A, Mondal MS, Suganuma T, Matsukura S, Kangawa K, Nakazato M. Ghrelin, a novel growth hormone-releasing acylated peptide, is synthesized in a distinct endocrine cell type in the gastrointestinal tracts of rats and humans. Endocrinology 2000a; 141:4255-4261.

Date Y, Murakami N, Kojima M, Kuroiwa T, Matsukura S, Kangawa K, Nakazato M. Central effects of a novel acylated peptide, ghrelin, on growth hormone release in rats. Biochem Biophys Res Commun 2000b; 275:477-480.

Date Y, Murakami N, Toshinai K, Matsukura S, Niijima A, Matsuo H, Kangawa K, Nakazato M. The role of the gastric afferent vagal nerve in ghrelin-induced feeding and growth hormone secretion in rats. Gastroenterology 2002; 123: 1120-1128.

Dornonville dlC, Bjorkqvist M, Sandvik AK, Bakke I, Zhao CM, Chen D, Hakanson R. A-like cells in the rat stomach contain ghrelin and do not operate under gastrin control. Regul Pept 2001; 99:141-150.

Gauna C, Meyler FM, Janssen JA, Delhanty PJ, Abribat T, van Koetsveld P, Hofland LJ, Broglio F, Ghigo E, van der Lely, A. J. Administration of acylated ghrelin reduces insulin sensitivity, whereas the combination of acylated plus unacylated ghrelin strongly improves insulin sensitivity. J Clin Endocrinol Metab 2004; 89:5035-5042.

Gauna C, Delhanty PJ, Hofland LJ, Janssen JA" Broglio F, Ross RJ, Ghigo E, van der Lely AJ. Ghrelin stimulates, whereas des-octanoyl ghrelin inhibits, glucose output by primary hepatocytes. J Clin Endocrinol Metab 2005; 90: 1055-1060.

Gauna C, Kiewiet RM, Janssen JA, van de, ZB, Delhanty P, Ghigo E, Hofland LJ, Themmen AP, van der Lely AJ. Unacylated ghrelin acts as a potent insulin-secretagogue in glucose-stimulated conditions. Am J Physiol Endocrinol Metab 2007.

Geisler S, Heilmann H, Veh RW. An optimized method for simultaneous demonstration of neurons and myelinated fiber tracts for delineation of individual trunco- and palliothalamic nuclei in the mammalian brain. Histochem Cell Biol 2002; 117:69-79.

Geliebter A. Gastric distension and gastric capacity in relation to food intake in humans. Physiol Behav 1988; 44: 665-668.

Halaas JL, Boozer C, Blair-West J, Fidahusein N, Denton DA, Friedman JM,: Physiological response to long-term peripheral and central leptin infusion in lean and obese mice. Proc Natl Acad Sci 1997; 94:8878-8883.

Heijboer AC, van den Hoek AM, Parlevliet ET, Havekes LM, Romijn JA, Pijl H, Corssmit EP. Ghrelin differentially affects hepatic and peripheral insulin sensitivity in mice. Diabetologia 2006.

Hewson AK, Dickson SL. Systemic administration of ghrelin induces Fos and Egr-1 proteins in the hypothalamic arcuate nucleus of fasted and fed rats. J Neuroendocrinol 2000; 12:1047-1049.

Hosoda H, Kojima M, Matsuo H, Kangawa K. Ghrelin and des-acyl ghrelin: two major forms of rat ghrelin peptide in gastrointestinal tissue. Biochem Biophys Res Commun 2000; 279:909-913.

Kihara N, Fujimura M, Yamamoto I, Itoh E, Inui A, Fujimiya M. Effects of central and peripheral urocortin on fed and fasted gastroduodenal motor activity in conscious rats. Am. J. Physiol Gastrointest. Liver Physiol 2001; 280:G406-G419.

Kobelt P, Tebbe JJ, Tjandra I, Stengel A, Bae HG, Andresen V, van dV I, Veh RW, Werner CR, Klapp BF, Wiedenmann B, Wang L, Tache Y, Monnikes H. CCK inhibits the orexigenic effect of peripheral ghrelin. Am J Physiol Regul Integr Comp Physiol 2005; 288:R751-R758.

Kobelt P, Goebel M, Stengel A., Schmidtmann M., van dV I, Tebbe JJ, Veh RW, Klapp BF, Wiedenmann B, Wang L, Tache Y, Monnikes H. Bombesin but not amylin blocks the orexigenic effect of peripheral ghrelin. Am J Physiol Regul Integr Comp Physiol 2006.

Kojima M, Hosoda H, Date Y, Nakazato M, Matsuo H, Kangawa K. Ghrelin is a growth-hormone-releasing acylated peptide from stomach. Nature 1999; 402:656-660.

Kojima M, Kongawa K. Ghrelin: Structure and Function. Physiol Rev 2005; 85:495-522.

Matsuda K, Miura T, Kaiya H, Maruyama K, Shimakura SI., Uchiyama M, Kangawa K, Shioda, S. Regulation of food intake by acyl and des-acyl ghrelins in the goldfish. Peptides 2006.

Nagaya N, Kojima M, Uematsu M, Yamagishi M, Hosoda H, Oya H, Hayashi Y, Kangawa K. Hemodynamic and hormonal effects of human ghrelin in healthy volunteers. Am J Physiol Regul Integr Comp Physiol 2001; 280:R1483-R1487.

Nakazato M, Murakami N, Date Y, Kojima M, Matsuo H, Kangawa K, Matsukura S. A role for ghrelin in the central regulation of feeding. Nature 2001; 409:194-198.

Neary NM, Druce MR, Small CJ, Bloom SR. Acylated ghrelin stimulates food intake in the fed and fasted states but desacylated ghrelin has no effect. Gut 2006; 55:135.

Paxinos G, Watson C. The rat brain in stereotaxic coordinates. San Diego: Academic Press, 1997.

Poitras P, Tasse D, Laprise P. Stimulation of motilin release by bombesin in dogs. Am J Physiol 1983; 245:G249-G256.

Rodriguez-Membrilla A, Martinez V, Vergara P. Peripheral and central cholecystokinin receptors regulate postprandial intestinal motility in the rat. J. Pharmacol. Exp Ther 1995; 275:486-493.

Ruter J, Kobelt P, Tebbe JJ, Avsar Y, Veh R, Wang L, Klapp BF, Wiedenmann B, Tache Y, Monnikes H. Intraperitoneal injection of ghrelin induces Fos expression in the paraventricular nucleus of the hypothalamus in rats. Brain Res 2003; 991:26-33.

Sagar SM, Sharp FR, Curran T. Expression of c-fos protein in brain: metabolic mapping at the cellular level. Science 1988; 240:1328-1331.

Seoane LM, Tovar S, Baldelli R, Arvat E, Ghigo E, Casanueva FF, Dieguez C. Ghrelin elicits a marked stimulatory effect on GH secretion in freely-moving rats. Eur J Endocrinol 2000; 143:R7-R9.

Takahashi LK. Role of CRF(1) and CRF(2) receptors in fear and anxiety. Neurosci Biobehav Rev 2001; 25:627-636.

Tassone F, Broglio F, Destefanis S, Rovere S, Benso A, Gottero C, Prodam F, Rossetto R, Gauna C, van der Leiy AJ, Ghigo E, Maccario M. Neuroendocrine and metabolic effects of acute ghrelin administration in human obesity. J Clin Endocrinol Metab 2003; 88:5478-5483.

Thomas GB, Fairhall KM, Robinson IC. Activation of the hypothalamo-pituitary-adrenal axis by the growth hormone (GH) secretagogue, GH-releasing peptide-6, in rats. Endocrinology 1997; 138:1585-1591.

Tolle V, Zizzari P, Tomasetto C, Rio MC, Epelbaum J, Bluet-Pajot MT. In vivo and in vitro effects of ghrelin/motilin-related peptide on growth hormone secretion in the rat. Neuroendocrinology 2001; 73:54-61.

Toshinai K, Yamaguchi H, Sun Y, Smith RG, Yamanaka A, Sakurai T, Date Y, Mondal MS, Shimbara T, Kawagoe T, Murakami N, Miyazato M, Kangawa K, Nakazato M. Desacyl Ghrelin Induces Food Intake by a Mechanism Independent of the Growth Hormone Secretagogue Receptor. Endocrinology 2006.

Tschop M, Smiley DL, Heiman ML. Ghrelin induces adiposity in rodents. Nature 2000; 407:908-913.

Vestergaard ET, Hansen TK, Gormsen, LC, Jakobsen P, Moller N, Christiansen JS, Jorgensen JO. Constant intravenous ghrelin infusion in healthy young men: Clinical pharmacokinetics and metabolic effects. Am J Physiol Endocrinol Metab 2007.

Wang L, Saint-Pierre DH, Tache Y. Peripheral ghrelin selectively increases Fos expression in neuropeptide Y - synthesizing neurons in mouse hypothalamic arcuate nucleus. Neurosci Lett 2002; 325:47-51.

West DB, Fey D, Woods SC. Cholecystokinin persistently suppresses meal size but not food-intake in free-feeding rats. American Journal of Physiology: Regulatory, Integrative and Comparative Physiology 1984; 246: R 776-787.

Wren AM, Small CJ, Ward HL, Murphy KG, Dakin CL, Taheri S, Kennedy AR, Roberts GH, Morgan DG, Ghatei MA, Bloom SR. The novel hypothalamic peptide ghrelin stimulates food intake and growth hormone secretion. Endocrinology 2000; 141:4325-4328.

Wren AM, Small CJ, Abbott CR, Dhillo WS, Seal LJ, Cohen MA, Batterham RL, Taheri S, Stanley SA, Ghatei MA, Bloom SR. Ghrelin causes hyperphagia and obesity in rats. Diabetes 2001a; 50:2540-2547.

Wren AM, Seal LJ, Cohen MA, Brynes AE, Frost GS, Murphy KG, Dhillo WS, Ghatei MA, Bloom SR. Ghrelin enhances appetite and increases food intake in humans. J Clin Endocrinol Metab 2001b; 86:5992.

Yoshimoto A, Mori K, Sugawara A et al. Plasma ghrelin and desacyl ghrelin concentrations in real failure. J Am Soc Nephrol 2002; 13:2748-2752.

**Claims**

1. A use of a desacyl ghrelin or an active fragment thereof for the preparation of a pharmaceutical composition for the treatment of hyperphagia in a subject in need thereof.

2. The use according to claim 1, wherein the desacyl ghrelin comprises a peptide having an amino acid sequence according to any of SEQ ID NO:1 to SEQ ID NO:21.

3. The use according to claim 1 or 2, wherein the active fragment of desacyl ghrelin comprises a peptide having an amino acid sequence according to any of SEQ ID NO:22 to SEQ ID NO:25.

4. The use according to any of the preceding claims, wherein hyperphagia is associated with body weight gain and/or obesity.

5. The use according to any of the preceding claims, wherein hyperphagia is associated with a central nervous disorder.

6. The use according to any of claims 1 to 4, wherein hyperphagia is associated with a disorder selected from a genetically disposed obesity, Prader-Labhart-Willi-Fanconi syndrome, Laurence-Moon-Biedl syndrome, Fröhlich's syndrome, central Cushing's syndrome, and hyperphagic short stature syndrome.

7. The use according to claim 6, wherein the genetically disposed obesity is associated with a MC4R gene mutation.

8. The use according to any of the preceding claims, wherein the subject has a body mass index BMI of> 30, preferably > 40.

9. The use according to any of the preceding claims, wherein the subject is an adult.

10. The use according to any of the preceding claims, wherein the pharmaceutical composition is comprised by a pharmaceutical dosage form selected from an injection, a tablet, a capsule, a caplet, a suppository, an elixier, an ointment, and a patch.

11. The use according to claim 10, wherein the pharmaceutical dosage form is a controlled-release dosage form, preferably a sustained-release dosage form.

Fig.1

Fig. 2.

Fig. 3.

**Fig. 4**

Fig. 5.

**Fig. 6**

European Patent
Office

**EUROPEAN SEARCH REPORT**

Application Number

EP 07 02 3373

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | ASAKAWA ET AL: "Stomach regulates energy balance via acylated ghrelin and desacyl ghrelin" GUT, vol. 54, 2005, pages 18-24, XP002480777 * See pages 23-24 (Discussion) * | 1-11 | INV. A61K38/22 A61P3/04 |
| Y,D | CHEN ET AL: "Des-acyl ghrelin acts by CRF type 2 receptors to disrupt fasted stomach motility in conscious rats" GASTROENTEROLOGY, vol. 129, 2005, pages 8-25, XP005313821 * See page 23, left column (end of the first paragraph / anorectic effects) * | 1-11 | |
| A | EBAL ET AL: "Effect of a lipid-enriched diet on body composition and some regulatory hormones of food intake in growing rats" MASSON FR (ABSTRACT ONLY), vol. 68, October 2007 (2007-10), pages 1-3, XP002481176 Retrieved from the Internet: URL:www.masson.fr/masson/portal/bookmark?Global=1&Page=18&MenuIdSelected=106&MenuItemSelected=0&MenuSupportSelected=0&CodeProduct4=180&CodeRevue4=AN&Path=REVUE/AN/2007/68/5/ARTICLE11943360076.xml&Locations=> [retrieved on 2008-05-19] * See Abstract (Conclusion) * | 1-11 | TECHNICAL FIELDS SEARCHED (IPC)  A61K A61P |
| P,Y | NONOGAKI: "Ghrelin and feedback systems" VITAMINS AND HORMONES, vol. 77, 2008, pages 149-170, XP008091705 * See pages 159-161 (item IV), and page 162 (last paragraph); Available online 4 November 2007 -> normal prior art * | 1-11 | |

-/--

The present search report has been drawn up for all claims

| Place of search | Date of completion of the search | Examiner |
|---|---|---|
| Berlin | 21 May 2008 | Korsner, Sven-Erik |

EPO FORM 1503 03.82 (P04C01)

## EUROPEAN SEARCH REPORT

**European Patent Office**

Application Number

EP 07 02 3373

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,Y | QADER ET AL: "Proghrelin-derived peptides influence the secretion of insulin, glucagon, pancreatic polypeptide and somatostatin: A study on isolated islets from mouse and rat pancreas" REGULATORY PEPTIDES, vol. 146, 2008, pages 230-237, XP022422007 * See page 233 (desacyl ghrelin) and page 236 (end of the Discussion); Available online 18 September 2007 -> normal prior art * ----- | 1-11 | |

TECHNICAL FIELDS SEARCHED (IPC)

The present search report has been drawn up for all claims

| Place of search | Date of completion of the search | Examiner |
|---|---|---|
| Berlin | 21 May 2008 | Korsner, Sven-Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6967237 B

- WO 03051389 A

**Non-patent literature cited in the description**

- **ANDO T ; RIVIER J ; YANAIHARA H ; ARIMURA A.** Peripheral corticotropin-releasing factor mediates the elevation of plasma IL-6 by immobilization stress in rats. *Am J Physiol,* 1998, vol. 275, R1461-R1467
- **ARNOLD M ; MURA A ; LANGHANS W ; GEARY N.** Gut vagal afferents are not necessary for the eating-stimulatory effect of intraperitoneally injected ghrelin in the rat. *J Neurosc,* 2006, vol. 26, 11052-11060
- **ARVAT E ; MACCARIO M ; DI VITO L ; BROGLIO F ; BENSO A ; GOTTERO C ; PAPOTTI M ; MUCCIOLI G ; DIEGUEZ C ; CASANUEVA FF.** Endocrine activities of ghrelin, a natural growth hormone secretagogue (GHS), in humans: comparison and interactions with hexarelin, a nonnatural peptidyl GHS, and GH-releasing hormone. *J Clin Endocrinol Metab,* 2001, vol. 86, 1169-1174
- **ASAKAWA A ; INUI A ; KAGA T ; YUZURIHA H ; NAGATA T ; FUJIMIYA M ; KATSUURA G ; MAKINO S ; FUJINO MA ; KASUGA M.** A role of ghrelin in neuroendocrine and behavioral responses to stress in mice. *Neuroendocrinology,* 2001, vol. 74, 143-147
- **ASAKAWA A ; INUI A ; KAGA T ; YUZURIHA H ; NAGATA T ; UENO N ; MAKINO S ; FUJIMIYA M ; NIIJIMA A ; FUJINO MA.** Ghrelin is an appetite-stimulatory signal from stomach with structural resemblance to motilin. *Gastroenterology,* 2001, vol. 120, 337-345
- **ASAKAWA A ; INUI A ; FUJIMIYA M ; SAKAMAKI R ; SHINFUKU N ; UETA Y ; MEGUID MM ; KASUGA M.** Stomach regulates energy balance via acylated ghrelin and desacyl ghrelin. *Gut,* 2005, vol. 54, 18-24
- **BALE TL ; VALE WW.** CRF and CRF receptors: role in stress responsivity and other behaviors. *Annu Rev Pharmacol Toxicol,* 2004, vol. 44, 525-557
- **BANKS WA ; TSCHOP M ; ROBINSON SM ; HEIMAN ML.** Extent and direction of ghrelin transport across the blood-brain barrier is determined by its unique primary structure. *J Pharmacol Exp Ther,* 2002, vol. 302, 822-827

- **BROGLIO F ; ARVAT E ; BENSO A ; GOTTERO C ; MUCCIOLI G ; PAPOTTI M ; VAN DER LELY A. J. DEGHENGHI, R. ; GHIGO, E.** Ghrelin, a natural GH secretagogue produced by the stomach, induces hyperglycemia and reduces insulin secretion in humans. *J. Clin. Endocrinol. Metab,* 2001, vol. 86, 5083-5086
- **CHANG CP ; PEARSE RV ; O'CONNELL S ; ROSENFELD MG.** Identification of a seven transmembrane helix receptor for corticotropin-releasing factor and sauvagine in mammalian brain. *Neuron,* 1993, vol. 11, 1187-1195
- **CHEN CY ; MILLION M ; ADELSON DW ; MARTINEZ V ; RIVIER J ; TACHE Y.** Intracisternal urocortin inhibits vagally stimulated gastric motility in rats: role of CRF(2). *Br J Pharmacol,* 2002, vol. 136, 237-247
- **CHEN CY ; INUI A ; ASAKAWA A ; FUJINO K ; KATO I ; CHEN CC ; UENO N ; FUJIMIYA M.** Des-acyl Ghrelin Acts by CRF Type 2 Receptors to Disrupt Fasted Stomach Motility in Conscious Rats. *Gastroenterology,* 2005, vol. 129, 8-25
- **CHEN CY ; CHAO Y ; CHANG FY ; CHIEN EJ ; LEE SD ; DOONG, ML.** Intracisternal des-acyl ghrelin inhibits food intake and non-nutrient gastric emptying in conscious rats. *Int J Mol Med,* 2005, vol. 16, 695-699
- **COSKUN T ; BOZKURT A ; ALICAN I ; OZKUTLU U ; KURTEL H ; YEGEN BC.** Pathways mediating CRF-induced inhibition of gastric emptying in rats. *Regul Pept,* 1997, vol. 69, 113-120
- **CRAWLEY JN ; BEINFELD MC.** Rapid development of tolerance to the behavioural actions of cholecystokinin. *Nature,* 1983, vol. 302, 703-706
- **DATE Y ; KOJIMA M ; HOSODA H ; SAWAGUCHI A ; MONDAL MS ; SUGANUMA T ; MATSUKURA S ; KANGAWA K ; NAKAZATO M.** Ghrelin, a novel growth hormone-releasing acylated peptide, is synthesized in a distinct endocrine cell type in the gastrointestinal tracts of rats and humans. *Endocrinology,* 2000, vol. 141, 4255-4261

- **DATE Y ; MURAKAMI N ; KOJIMA M ; KUROIWA T ; MATSUKURA S ; KANGAWA K ; NAKAZATO M.** Central effects of a novel acylated peptide, ghrelin, on growth hormone release in rats. *Biochem Biophys Res Commun,* vol. 275, 477-480
- **DATE Y ; MURAKAMI N ; TOSHINAI K ; MATSU-KURA S ; NIIJIMA A ; MATSUO H ; KANGAWA K ; NAKAZATO M.** The role of the gastric afferent vagal nerve in ghrelin-induced feeding and growth hormone secretion in rats. *Gastroenterology,* vol. 123, 1120-1128
- **DORNONVILLE DLC ; BJORKQVIST M ; SAND-VIK AK ; BAKKE I ; ZHAO CM ; CHEN D ; HAKAN-SON R.** A-like cells in the rat stomach contain ghrelin and do not operate under gastrin control. *Regul Pept,* 2001, vol. 99, 141-150
- **GAUNA C ; MEYLER FM ; JANSSEN JA ; DEL-HANTY PJ ; ABRIBAT T ; VAN KOETSVELD P ; HOFLAND LJ ; BROGLIO F ; GHIGO E ; VAN DER LELY, A. J.** Administration of acylated ghrelin reduces insulin sensitivity, whereas the combination of acylated plus unacylated ghrelin strongly improves insulin sensitivity. *J Clin Endocrinol Metab,* 2004, vol. 89, 5035-5042
- **GAUNA C ; DELHANTY PJ ; HOFLAND LJ ; JANSSEN JA ; BROGLIO F ; ROSS RJ ; GHIGO E ; VAN DER LELY AJ.** Ghrelin stimulates, whereas des-octanoyl ghrelin inhibits, glucose output by primary hepatocytes. *J Clin Endocrinol Metab,* 2005, vol. 90, 1055-1060
- **GAUNA C ; KIEWIET RM ; JANSSEN JA ; VAN DE, ZB ; DELHANTY P ; GHIGO E ; HOFLAND LJ.** Themmen AP, van der Lely AJ. Unacylated ghrelin acts as a potent insulin-secretagogue in glucose-stimulated conditions. *Am J Physiol Endocrinol Metab,* 2007
- **GEISLER S ; HEILMANN H ; VEH RW.** An optimized method for simultaneous demonstration of neurons and myelinated fiber tracts for delineation of individual trunco- and palliothalamic nuclei in the mammalian brain. *Histochem Cell Biol,* 2002, vol. 117, 69-79
- **GELIEBTER A.** Gastric distension and gastric capacity in relation to food intake in humans. *Physiol Behav,* 1988, vol. 44, 665-668
- **HALAAS JL ; BOOZER C ; BLAIR-WEST J ; FIDA-HUSEIN N ; DENTON DA ; FRIEDMAN JM.** Physiological response to long-term peripheral and central leptin infusion in lean and obese mice. *Proc Natl Acad Sci,* 1997, vol. 94, 8878-8883
- **HEIJBOER AC ; VAN DEN HOEK AM ; PARLEV-LIET ET ; HAVEKES LM ; ROMIJN JA ; PIJL H.** Corssmit EP. Ghrelin differentially affects hepatic and peripheral insulin sensitivity in mice. *Diabetologia,* 2006
- **HEWSON AK ; DICKSON SL.** Systemic administration of ghrelin induces Fos and Egr-1 proteins in the hypothalamic arcuate nucleus of fasted and fed rats. *J Neuroendocrinol,* 2000, vol. 12, 1047-1049
- **HOSODA H ; KOJIMA M ; MATSUO H ; KAN-GAWA K.** Ghrelin and des-acyl ghrelin: two major forms of rat ghrelin peptide in gastrointestinal tissue. *Biochem Biophys Res Commun,* 2000, vol. 279, 909-913
- **KIHARA N ; FUJIMURA M ; YAMAMOTO I ; ITOH E ; INUI A ; FUJIMIYA M.** Effects of central and peripheral urocortin on fed and fasted gastroduodenal motor activity in conscious rats. *Am. J. Physiol Gastrointest. Liver Physiol,* 2001, vol. 280, G406-G419
- **KOBELT P ; TEBBE JJ ; TJANDRA I ; STENGEL A ; BAE HG ; ANDRESEN V ; VAN DV I ; VEH RW ; WERNER CR ; KLAPP BF.** CCK inhibits the orexigenic effect of peripheral ghrelin. *Am J Physiol Regul Integr Comp Physiol,* 2005, vol. 288, R751-R758
- **KOBELT P ; GOEBEL M ; STENGEL A. ; SCHMIDTMANN M. ; VAN DV I ; TEBBE JJ ; VEH RW ; KLAPP BF ; WIEDENMANN B ; WANG L.** Bombesin but not amylin blocks the orexigenic effect of peripheral ghrelin. *Am J Physiol Regul Integr Comp Physiol,* 2006
- **KOJIMA M ; HOSODA H ; DATE Y ; NAKAZATO M ; MATSUO H ; KANGAWA K.** Ghrelin is a growth-hormone-releasing acylated peptide from stomach. *Nature,* 1999, vol. 402, 656-660
- **KOJIMA M ; KONGAWA K.** Ghrelin: Structure and Function. *Physiol Rev,* 2005, vol. 85, 495-522
- **MATSUDA K ; MIURA T ; KAIYA H ; MARUYAMA K ; SHIMAKURA SI. ; UCHIYAMA M ; KANGAWA K ; SHIODA, S.** Regulation of food intake by acyl and des-acyl ghrelins in the goldfish. *Peptides,* 2006
- **NAGAYA N ; KOJIMA M ; UEMATSU M ; YAMAG-ISHI M ; HOSODA H ; OYA H ; HAYASHI Y ; KAN-GAWA K.** Hemodynamic and hormonal effects of human ghrelin in healthy volunteers. *Am J Physiol Regul Integr Comp Physiol,* 2001, vol. 280, R1483-R1487
- **NAKAZATO M ; MURAKAMI N ; DATE Y ; KOJIMA M ; MATSUO H ; KANGAWA K ; MATSUKURA S.** A role for ghrelin in the central regulation of feeding. *Nature,* 2001, vol. 409, 194-198
- **NEARY NM ; DRUCE MR ; SMALL CJ ; BLOOM SR.** Acylated ghrelin stimulates food intake in the fed and fasted states but desacylated ghrelin has no effect. *Gut,* 2006, vol. 55, 135
- The rat brain in stereotaxic coordinates. **PAXINOS G ; WATSON C.** The rat brain in stereotaxic coordinates. Academic Press, 1997
- **POITRAS P ; TASSE D ; LAPRISE P.** Stimulation of motilin release by bombesin in dogs. *Am J Physiol,* 1983, vol. 245, G249-G256

- **RODRIGUEZ-MEMBRILLA A ; MARTINEZ V ; VERGARA P.** Peripheral and central cholecystokinin receptors regulate postprandial intestinal motility in the rat. *J. Pharmacol. Exp Ther,* 1995, vol. 275, 486-493
- **RUTER J ; KOBELT P ; TEBBE JJ ; AVSAR Y ; VEH R ; WANG L ; KLAPP BF ; WIEDENMANN B ; TACHE Y ; MONNIKES H.** Intraperitoneal injection of ghrelin induces Fos expression in the paraventricular nucleus of the hypothalamus in rats. *Brain Res,* 2003, vol. 991, 26-33
- **SAGAR SM ; SHARP FR ; CURRAN T.** Expression of c-fos protein in brain: metabolic mapping at the cellular level. *Science,* 1988, vol. 240, 1328-1331
- **SEOANE LM ; TOVAR S ; BALDELLI R ; ARVAT E ; GHIGO E ; CASANUEVA FF ; DIEGUEZ C.** Ghrelin elicits a marked stimulatory effect on GH secretion in freely-moving rats. *Eur J Endocrinol,* 2000, vol. 143, R7-R9
- **TAKAHASHI LK.** Role of CRF(1) and CRF(2) receptors in fear and anxiety. *Neurosci Biobehav Rev,* 2001, vol. 25, 627-636
- **TASSONE F ; BROGLIO F ; DESTEFANIS S ; ROVERE S ; BENSO A ; GOTTERO C ; PRODAM F ; ROSSETTO R ; GAUNA C ; VAN DER LEIY AJ.** Neuroendocrine and metabolic effects of acute ghrelin administration in human obesity. *J Clin Endocrinol Metab,* 2003, vol. 88, 5478-5483
- **THOMAS GB ; FAIRHALL KM ; ROBINSON IC.** Activation of the hypothalamo-pituitary-adrenal axis by the growth hormone (GH) secretagogue, GH-releasing peptide-6, in rats. *Endocrinology,* 1997, vol. 138, 1585-1591
- **TOLLE V ; ZIZZARI P ; TOMASETTO C ; RIO MC ; EPELBAUM J ; BLUET-PAJOT MT.** In vivo and in vitro effects of ghrelin/motilin-related peptide on growth hormone secretion in the rat. *Neuroendocrinology,* 2001, vol. 73, 54-61
- **TOSHINAI K ; YAMAGUCHI H ; SUN Y ; SMITH RG ; YAMANAKA A ; SAKURAI T ; DATE Y ; MONDAL MS ; SHIMBARA T ; KAWAGOE T.** Desacyl Ghrelin Induces Food Intake by a Mechanism Independent of the Growth Hormone Secretagogue Receptor. *Endocrinology,* 2006
- **TSCHOP M ; SMILEY DL ; HEIMAN ML.** Ghrelin induces adiposity in rodents. *Nature,* 2000, vol. 407, 908-913
- **VESTERGAARD ET ; HANSEN TK ; GORMSEN, LC ; JAKOBSEN P ; MOLLER N ; CHRISTIANSEN JS ; JORGENSEN JO.** Constant intravenous ghrelin infusion in healthy young men: Clinical pharmacokinetics and metabolic effects. *Am J Physiol Endocrinol Metab,* 2007
- **WANG L ; SAINT-PIERRE DH ; TACHE Y.** Peripheral ghrelin selectively increases Fos expression in neuropeptide Y - synthesizing neurons in mouse hypothalamic arcuate nucleus. *Neurosci Lett,* 2002, vol. 325, 47-51
- **WEST DB ; FEY D ; WOODS SC.** Cholecystokinin persistently suppresses meal size but not food-intake in free-feeding rats. *American Journal of Physiology: Regulatory, Integrative and Comparative Physiology,* 1984, vol. 246, R 776-787
- **WREN AM ; SMALL CJ ; WARD HL ; MURPHY KG ; DAKIN CL ; TAHERI S ; KENNEDY AR ; ROBERTS GH ; MORGAN DG ; GHATEI MA.** The novel hypothalamic peptide ghrelin stimulates food intake and growth hormone secretion. *Endocrinology,* 2000, vol. 141, 4325-4328
- **WREN AM ; SMALL CJ ; ABBOTT CR ; DHILLO WS ; SEAL LJ ; COHEN MA ; BATTERHAM RL ; TAHERI S ; STANLEY SA ; GHATEI MA.** Ghrelin causes hyperphagia and obesity in rats. *Diabetes,* 2001, vol. 50, 2540-2547
- **WREN AM ; SEAL LJ ; COHEN MA ; BRYNES AE ; FROST GS ; MURPHY KG ; DHILLO WS ; GHATEI MA ; BLOOM SR.** Ghrelin enhances appetite and increases food intake in humans. *J Clin Endocrinol Metab,* 2001, vol. 86, 5992
- **YOSHIMOTO A ; MORI K ; SUGAWARA A et al.** Plasma ghrelin and desacyl ghrelin concentrations in real failure. *J Am Soc Nephrol,* 2002, vol. 13, 2748-2752

SEQUENCE LISTING

<110> Charité - Universitätsmedizin Berlin

<120> Therapeutic Use of Desacyl Ghrelin

<130> FB19064

<160> 25

<170> PatentIn version 3.3

<210> 1
<211> 28
<212> PRT
<213> Homo sapiens

<400> 1

Gly Ser Ser Phe Leu Ser Pro Glu His Gln Arg Val Gln Gln Arg Lys
1               5                   10                  15

Glu Ser Lys Lys Pro Pro Ala Lys Leu Gln Pro Arg
            20                  25

<210> 2
<211> 28
<212> PRT
<213> Macaca mulatta

<400> 2

Gly Ser Ser Phe Leu Ser Pro Glu His Gln Arg Ala Gln Gln Arg Lys
1               5                   10                  15

Glu Ser Lys Lys Pro Pro Ala Lys Leu Gln Pro Arg
            20                  25

<210> 3
<211> 28
<212> PRT
<213> Mus sp.

<400> 3

Gly Ser Ser Phe Leu Ser Pro Glu His Gln Lys Ala Gln Gln Arg Lys
1               5                   10                  15

Glu Ser Lys Lys Pro Pro Ala Lys Leu Gln Pro Arg
            20                  25

<210> 4
<211> 28
<212> PRT
<213> Meriones unguiculatus

<400> 4

```
Gly Ser Ser Phe Leu Ser Pro Glu His Gln Lys Thr Gln Gln Arg Lys
1               5                   10                  15

Glu Ser Lys Lys Pro Pro Ala Lys Leu Gln Pro Arg
            20                  25


<210>  5
<211>  28
<212>  PRT
<213>  Rattus sp.

<400>  5

Gly Ser Ser Phe Leu Ser Pro Glu His Gln Lys Ala Gln Gln Arg Lys
1               5                   10                  15

Glu Ser Lys Lys Pro Pro Ala Lys Leu Gln Pro Arg
            20                  25


<210>  6
<211>  28
<212>  PRT
<213>  Canis sp.

<400>  6

Gly Ser Ser Phe Leu Ser Pro Glu His Gln Lys Leu Gln Gln Arg Lys
1               5                   10                  15

Glu Ser Lys Lys Pro Pro Ala Lys Leu Gln Pro Arg
            20                  25


<210>  7
<211>  28
<212>  PRT
<213>  Sus sp.

<400>  7

Gly Ser Ser Phe Leu Ser Pro Glu His Gln Lys Val Gln Gln Arg Lys
1               5                   10                  15

Glu Ser Lys Lys Pro Ala Ala Lys Leu Lys Pro Arg
            20                  25


<210>  8
<211>  27
<212>  PRT
<213>  Ovis sp.

<400>  8

Gly Ser Ser Phe Leu Ser Pro Glu His Gln Lys Leu Gln Arg Lys Glu
1               5                   10                  15
```

17

```
          Pro Lys Lys Pro Ser Gly Arg Leu Lys Pro Arg
                      20                  25


          <210>   9
          <211>   27
          <212>   PRT
          <213>   Bos sp.

          <400>   9

          Gly Ser Ser Phe Leu Ser Pro Glu His Gln Lys Leu Gln Arg Lys Glu
          1                   5                   10                  15


          Ala Lys Lys Pro Ser Gly Arg Leu Lys Pro Arg
                      20                  25


          <210>   10
          <211>   26
          <212>   PRT
          <213>   Gallus sp.

          <400>   10

          Gly Ser Ser Phe Leu Ser Pro Thr Tyr Lys Asn Ile Gln Gln Gln Lys
          1                   5                   10                  15


          Asp Thr Arg Lys Pro Thr Ala Arg Leu His
                      20                  25


          <210>   11
          <211>   26
          <212>   PRT
          <213>   Anas sp.

          <400>   11

          Gly Ser Ser Phe Leu Ser Pro Glu Phe Lys Lys Ile Gln Gln Gln Asn
          1                   5                   10                  15


          Asp Pro Thr Lys Thr Thr Ala Lys Ile His
                      20                  25


          <210>   12
          <211>   26
          <212>   PRT
          <213>   Dromaius sp.

          <400>   12

          Gly Ser Ser Phe Leu Ser Pro Asp Tyr Lys Lys Ile Gln Gln Arg Lys
          1                   5                   10                  15
```

```
Asp Pro Arg Lys Pro Thr Thr Lys Leu His
            20                  25


<210>  13
<211>  26
<212>  PRT
<213>  Anser sp.

<400>  13

Gly Ser Ser Phe Leu Ser Pro Glu Phe Lys Lys Ile Gln Gln Gln Asn
1               5                   10                  15


Asp Pro Ala Lys Ala Thr Ala Lys Ile His
            20                  25



<210>  14
<211>  28
<212>  PRT
<213>  Meleagris sp.

<400>  14

Gly Ser Ser Phe Leu Ser Pro Ala Tyr Lys Asn Ile Gln Gln Gln Lys
1               5                   10                  15


Asp Thr Arg Lys Pro Thr Ala Arg Leu His Pro Arg
            20                  25


<210>  15
<211>  23
<212>  PRT
<213>  Oncorhynchus mykiss 1

<400>  15

Gly Ser Ser Phe Leu Ser Pro Ser Gln Lys Pro Gln Val Arg Gln Gly
1               5                   10                  15


Lys Gly Lys Pro Pro Arg Val
            20


<210>  16
<211>  20
<212>  PRT
<213>  Oncorhynchus mykiss 2

<400>  16

Gly Ser Ser Phe Leu Ser Pro Ser Gln Lys Pro Gln Gly Lys Gly Lys
1               5                   10                  15


Pro Pro Arg Val
            20
```

```
<210>   17
<211>   21
<212>   PRT
<213>   Anguilla japonica

<400>   17

Gly Ser Ser Phe Leu Ser Pro Ser Gln Arg Pro Gln Gly Lys Asp Lys
1               5               10              15


Lys Pro Pro Arg Val
            20



<210>   18
<211>   19
<212>   PRT
<213>   Carassius auratus

<400>   18

Gly Thr Ser Phe Leu Ser Pro Ala Gln Lys Pro Gln Gly Arg Arg Pro
1               5               10              15


Pro Arg Met



<210>   19
<211>   19
<212>   PRT
<213>   Danio rerio

<400>   19

Gly Thr Ser Phe Leu Ser Pro Thr Gln Lys Pro Gln Gly Arg Arg Pro
1               5               10              15


Pro Arg Val



<210>   20
<211>   20
<212>   PRT
<213>   Tilapia sp.

<400>   20

Gly Ser Ser Phe Leu Ser Pro Ser Gln Lys Pro Gln Asn Lys Val Lys
1               5               10              15


Ser Ser Arg Ile
            20



<210>   21
<211>   28
```

```
<212>   PRT
<213>   Rana catesbeiana

<400>   21

Gly Leu Thr Phe Leu Ser Pro Ala Asp Met Gln Lys Ile Ala Glu Arg
1                   5                   10                  15


Gln Ser Gln Asn Lys Leu Arg His Gly Asn Met Asn
            20                  25



<210>   22
<211>   18
<212>   PRT
<213>   Homo sapiens

<400>   22

Gly Ser Ser Phe Leu Ser Pro Glu His Gln Arg Val Gln Gln Arg Lys
1                   5                   10                  15


Glu Ser



<210>   23
<211>   14
<212>   PRT
<213>   Homo sapiens

<400>   23

Gly Ser Ser Phe Leu Ser Pro Glu His Gln Arg Val Gln Gln
1                   5                   10



<210>   24
<211>   10
<212>   PRT
<213>   Homo sapiens, Rattus sp.

<400>   24

Gly Ser Ser Phe Leu Ser Pro Glu His Gln
1                   5                   10



<210>   25
<211>   5
<212>   PRT
<213>   Homo sapiens, Rattus sp.

<400>   25

Gly Ser Ser Phe Leu
1                   5
```